# EUROPEAN PATENT APPLICATION

(11) **EP 3 346 440 A1**
(43) Date of publication of application: **11.07.2018**
(21) Application number: 16851187.1
(22) Date of filing: 15.09.2016
(51) Int. Cl.: G06Q 50/24, A61J 3/00, G06F 3/048

(54) **INFORMATION PROCESSING DEVICE, METHOD AND PROGRAM**

(30) Priority: 29.09.2015 JP 2015190752
(71) Applicant: Sony Corporation, Tokyo 108-0075 (JP)
(72) Inventor: NAITO Kaori, Tokyo 108-0075 (JP); TAKAGI Yoshinori, Tokyo 108-0075 (JP); FUKUSHI Gakuho, Tokyo 108-0075 (JP)
(74) Representative: D Young & Co LLP
(86) International application number: PCT/JP2016/077222
(87) International publication number: WO 2017/057013

(57) **Abstract**

The present technology relates to an information processing apparatus and method as well as a program that make it possible to grasp information more rapidly.

A medication history display image has a medication history plot displaying region provided thereon, and medication history information is plot-displayed in a time series on a time axis in the medication history plot displaying region. At this time, medication history information of other dispensing dates is displayed in a display form according to a relation with medication history information of a current dispensing date. In particular, for example, in response to a relation to medication history information of the current dispensing date, same medicine information, contraindication-for-coadministration information, precaution-for-coadministration information or the like is displayed as the medication history information of the other dispensing dates. Consequently, it becomes possible to more rapidly and intuitively grasp necessary information. The present technology can be applied to an information processing apparatus.

## Description

### [Technical Field]

The present technology relates to an information processing apparatus and method as well as a program, and particularly to an information processing apparatus and method as well as a program by which information can be grasped rapidly.

### [Background Art]

In recent years, a system is being constructed in which prescriptions issued by doctors or drug notebooks issued by pharmacies are managed in the form of electronic data to improve the convenience or the efficiency.

For example, as a technology relating to medicines, a technology has been proposed in which, if a patient and prescription medicine names prescribed for the patient are inputted to a terminal apparatus in which information relating to prescription medicines, patient information such as personal information, medication histories and so forth of patients are recorded, then periods of use of all medicines prescribed for the patient are displayed in a graph on a calendar (for example, refer to PTL 1).

If such a technology as described above is utilized, then, for example, a doctor or a pharmacist can refer to the information relating to the medicines displayed in the form of a graph on a calendar to check the safety regarding coadministration of the medicines or give a guidance for medication to the patient.

### [Citation List]

### [Patent Literature]

[PTL 1]
JP 2004-185181A

### [Summary]

### [Technical Problem]

However, with the technology described above, although periods of use of all medicines prescribed for a patient are displayed in the form of a graph on a calendar, a doctor or a pharmacist who sees the display may not be able to rapidly grasp necessary information. For example, if periods of use of medicines are merely displayed, it is difficult for a doctor or a pharmacist to grasp a relation of a specific medicine to other medicines, and it is sometimes necessary to display detailed information or the like as occasion demands.

The present technology has been made in such a situation as described above and makes it possible to grasp information more rapidly.

### [Solution to Problem]

An information processing apparatus according to one aspect of the present technology includes a control unit configured to control such that pieces of medication history information recorded in an associated relation with personal identification information for identifying a patient are displayed in a lined up relation in a time series. The control unit controls such that the medication history information other than specific medication history information is displayed in a display form according to the specific medication history information of the medication history information.

The specific medication history information may be information of one or more medicines dispensed, prescribed or taken at a specific point of time.

The medication history information may include information relating to a medicine scheduled to be taken in the future by the patient.

A display form according to specific medication history information selected from within the medication history information may be a display form based on attribute information relating to coadministration of a medicine included in the specific medication history information and a medicine included in the medication history information other than the specific medication history information.

The attribute information may include an attribute relating to contraindication for coadministration and an attribute relating to precaution for coadministration.

Where a plurality of attributes different from each other are included in the same dispensing date, the control unit may be caused to control such that the attribute having a higher priority degree is displayed preferentially.

The control unit may be caused to control such that medication period information is displayed in a bar-like display as the medication history information other than the specific medication history information.

The medication period information may be information indicative of a medication period calculated from prescription information or dispensing information of the medication history information or a medication period during which a medicine of the medication history information is medicated actually.

The bar-like display may be a display indicative of a gradual decrease of a medicinal effect of a medicine or a medication amount of the medicine.

The control unit may be caused to control such that a remaining number of the medicines of the medication history information is calculated and displayed on the basis of prescription information or dispensing information of the medication history information or a medication period during which a medicine of the medication history information is medicated actually.

Where there is medication history information other than the specific medication history information for medication within a period that overlaps with a medication period of the specific medication history information, the control unit may be caused to control such that an alert is displayed.

The control unit may be caused to control such that the medication history information is plot-displayed at a position of a dispensing date of the medication history information on a time axis.

The control unit may be caused to control such that patient information associated with the personal identification information is displayed.

The patient information may be information indicative of a measurement value, an inspection value or a meal substance relating to a patient.

The control unit may be caused to control such that an alert is displayed on the basis of the medication history information and the patient information.

The control unit may be caused to control such that information relating to washout of a medicine of the medication history information is further displayed.

The information processing apparatus may further include a display unit configured to display the medication history information on the basis of display data.

The information processing apparatus may further include a recording unit configured to record the medication history information in association with the personal identification information.

An information processing method or a program according to one aspect of the present technology includes the steps of controlling such that pieces of medication history information recorded in an associated relation with personal identification information for identifying a patient are displayed in a lined up relation in a time series, and controlling such that the medication history information other than specific medication history information is displayed in a display form according to the specific medication history information of the medication history information.

In one aspect of the present technology, medication history information recorded in an associated relation with personal identification information for specifying a patient is controlled such that pieces of the medication history information other than specific medication history information from within the medication history information are displayed in a lined up relation in a time series in a display form according to the specific medication history information.

### [Advantageous Effect of Invention]

According to the one aspect of the present technology, information can be grasped more rapidly.

It is to be noted that the effect described here is not necessarily restrictive but may be any one of effects described herein.

### [Brief Description of Drawings]

[FIG. 1]

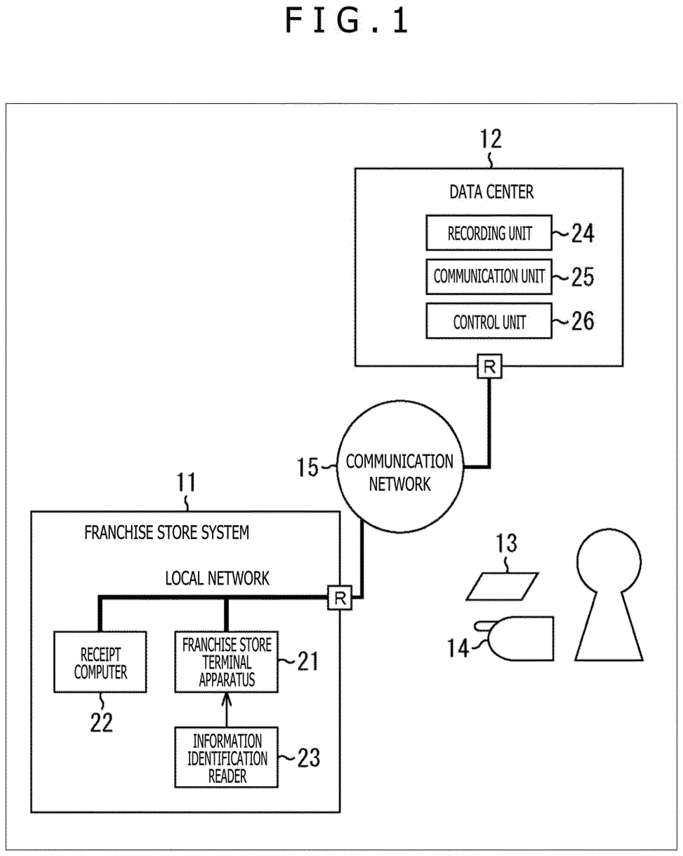

   FIG. 1 is a view depicting an example of a configuration of an information processing system.
[FIG. 2]

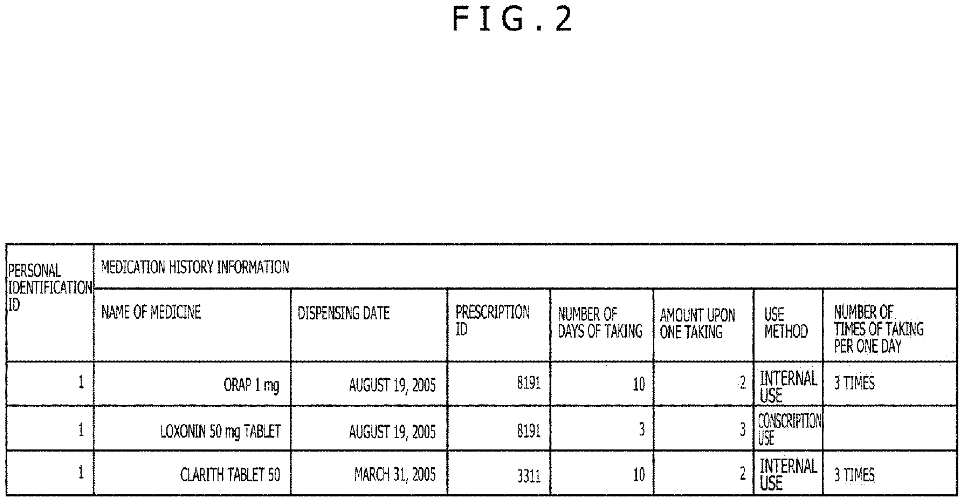

   FIG. 2 is a view depicting an example of dispensing history information.
[FIG. 3]

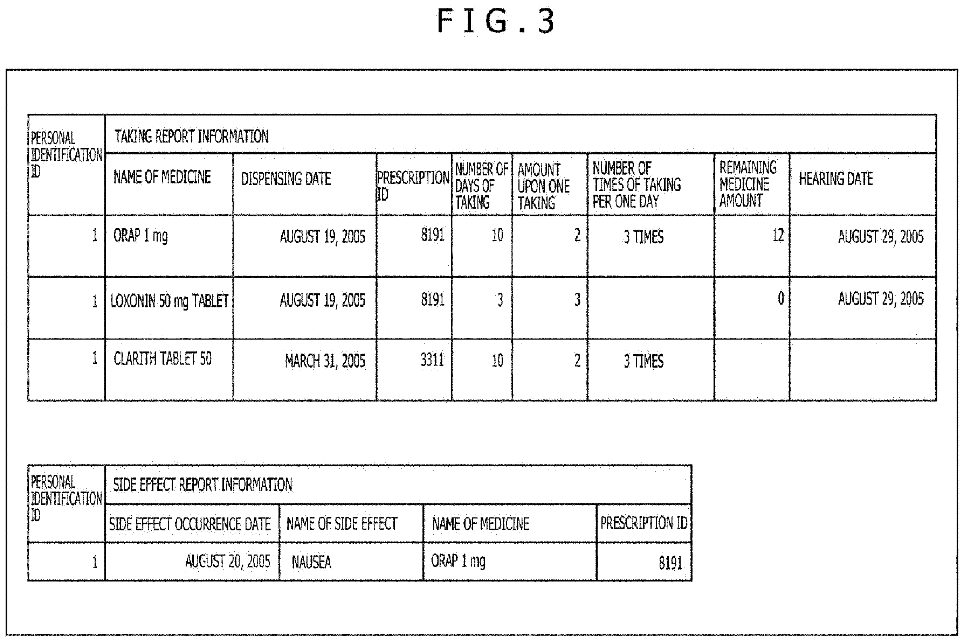

   FIG. 3 is a view depicting an example of taking report information and side effect report information.
[FIG. 4]

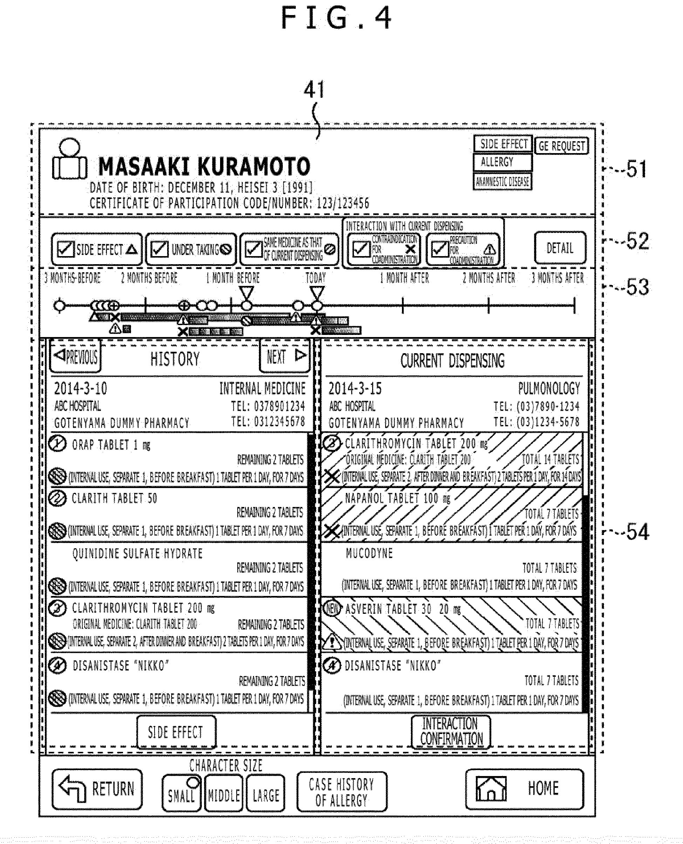

   FIG. 4 is a view depicting an example of a display of a medication history display image.
[FIG. 5]

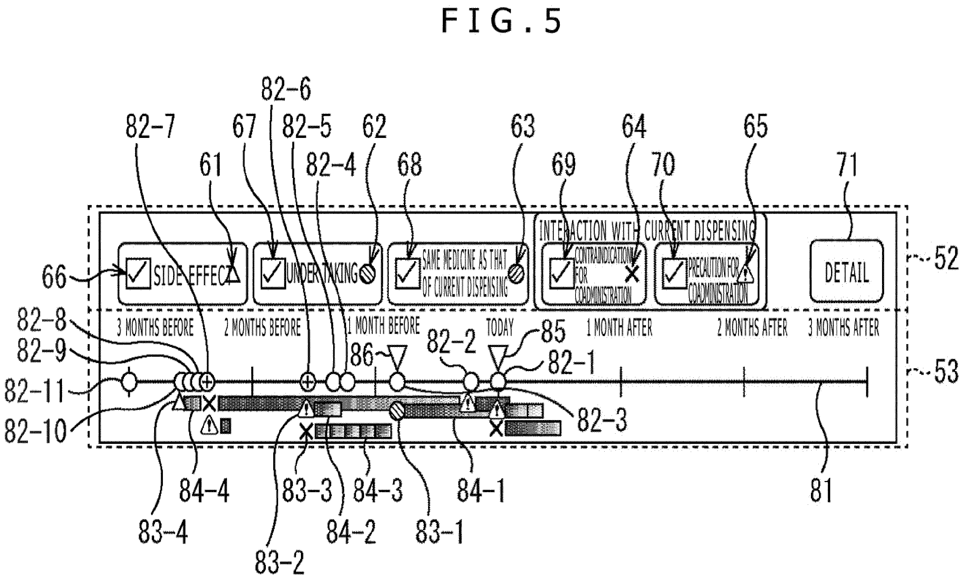

   FIG. 5 is a view depicting an example of a display of the medication history display image.
[FIG. 6]

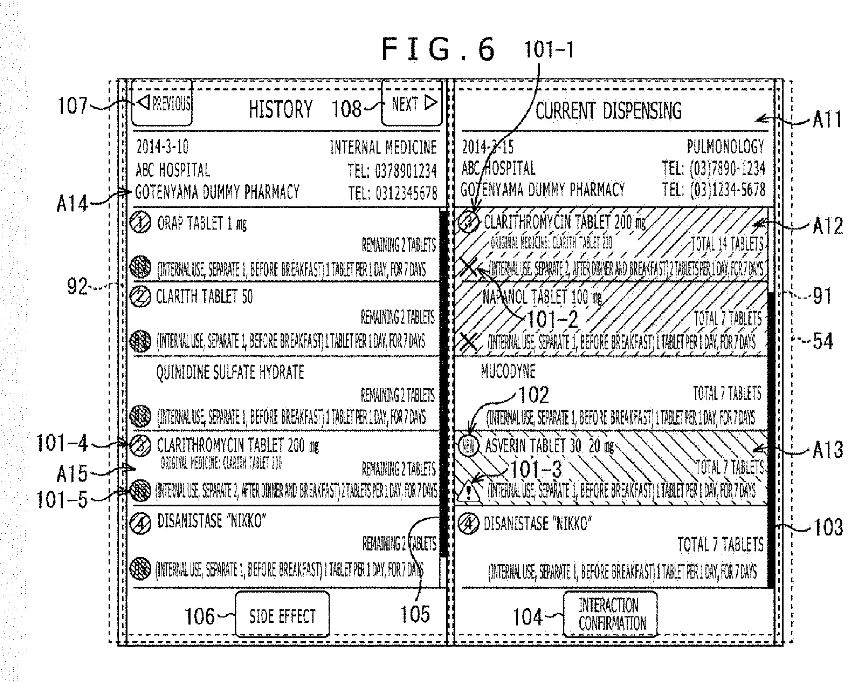

   FIG. 6 is a view depicting an example of another display of the medication history display image.
[FIG. 7]

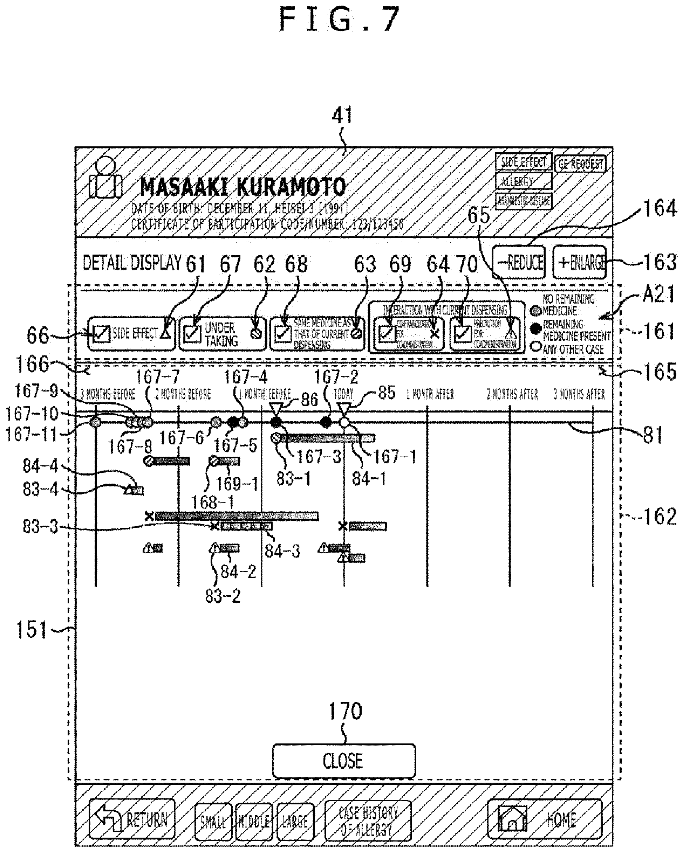

   FIG. 7 is a view depicting an example of a display of a detail display image.
[FIG. 8]

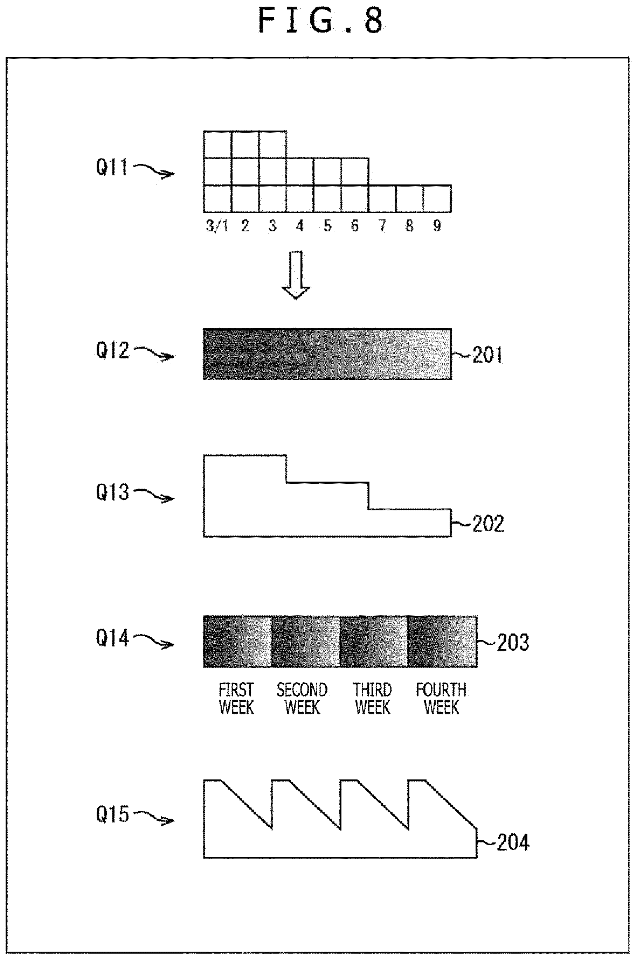

   FIG. 8 is a view illustrating medication schedule information.
[FIG. 9]

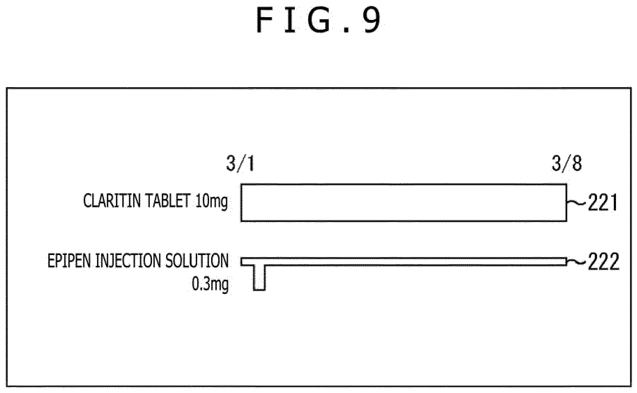

   FIG. 9 is a view illustrating medication schedule information.
[FIG. 10]

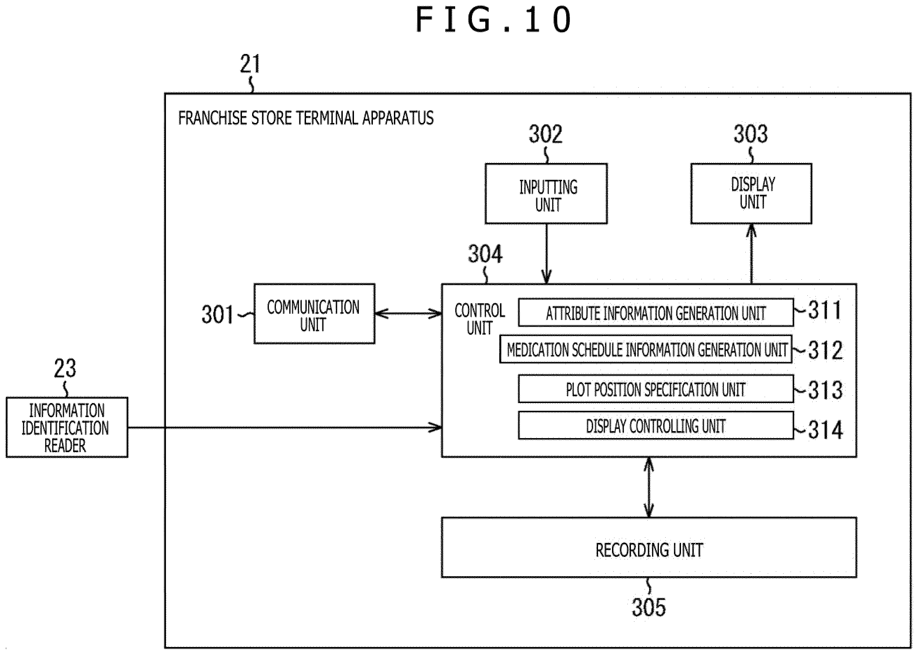

   FIG. 10 is a view depicting an example of a configuration of a franchise store terminal apparatus.
[FIG. 11]

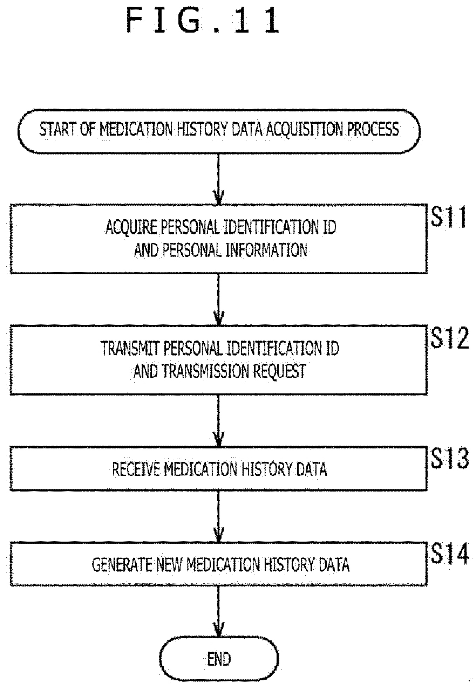

   FIG. 11 is a flow chart illustrating a medication history data acquisition process.
[FIG. 12]

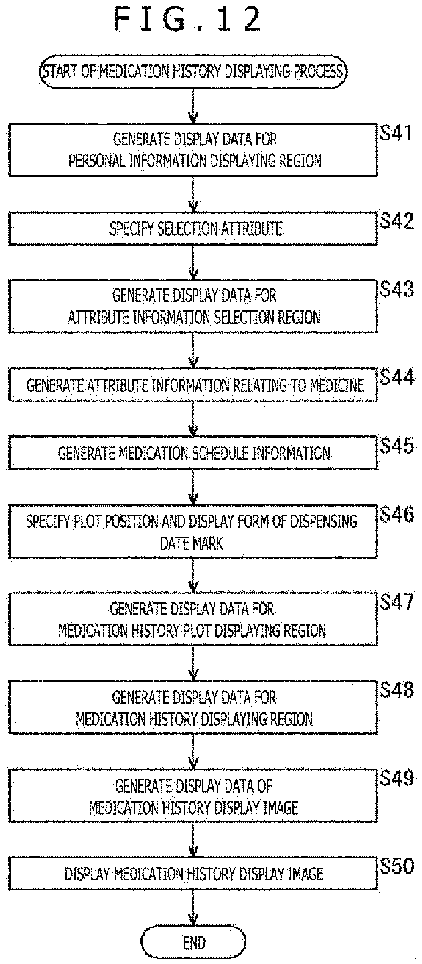

   FIG. 12 is a flow chart illustrating a medication history displaying process.
[FIG. 13]

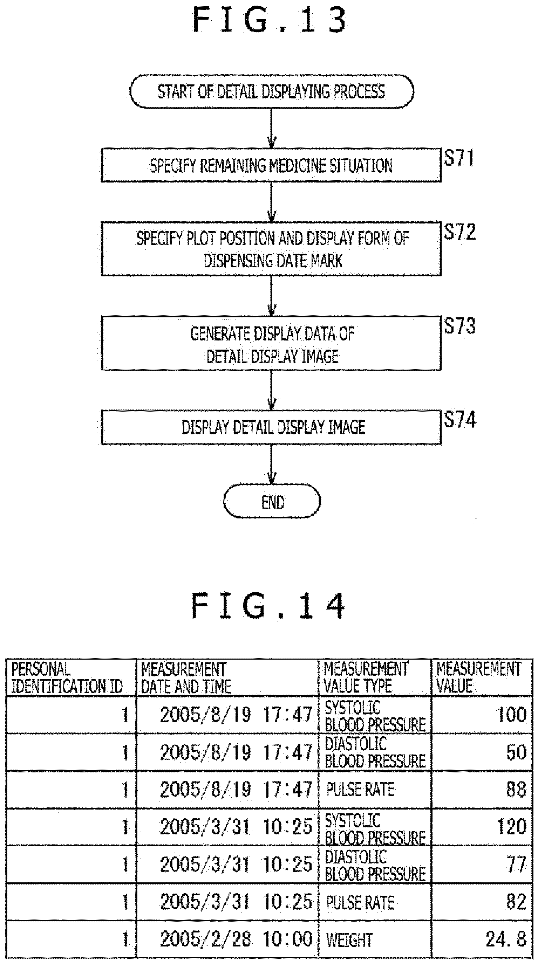

   FIG. 13 is a flow chart illustrating a detail displaying process.
[FIG. 14]
   FIG. 14 is a view depicting an example of patient information.
[FIG. 15]

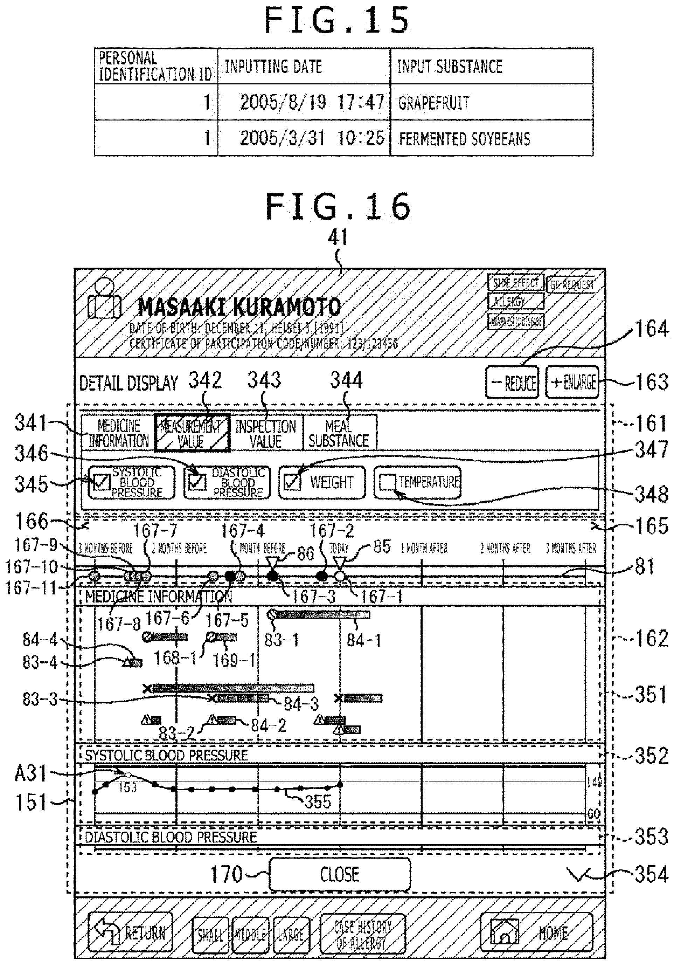

   FIG. 15 is a view depicting an example of patient information.
[FIG. 16]
   FIG. 16 is a view depicting an example of a display of a detail display image.
[FIG. 17]

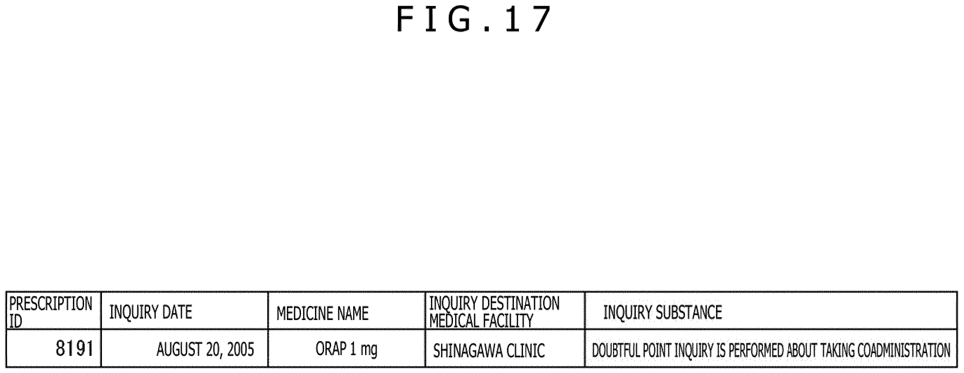

   FIG. 17 is a view depicting an example of information indicative of a result of doubtful point inquiry.
[FIG. 18]

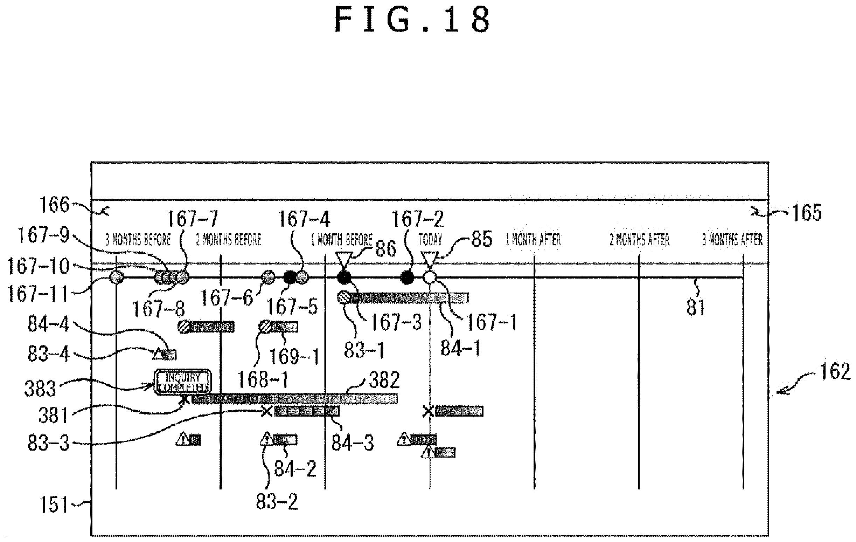

   FIG. 18 is a view depicting an example of a display of a detail display image.
[FIG. 19]

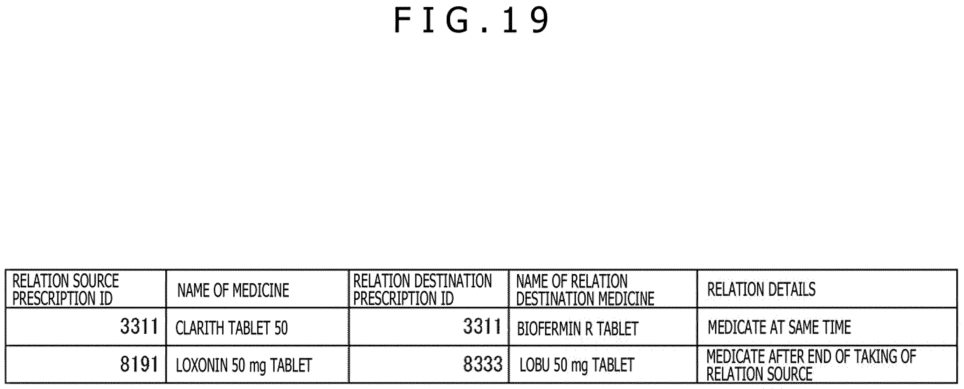

   FIG. 19 is a view depicting an example of information for specifying a relation between medicines.
[FIG. 20]

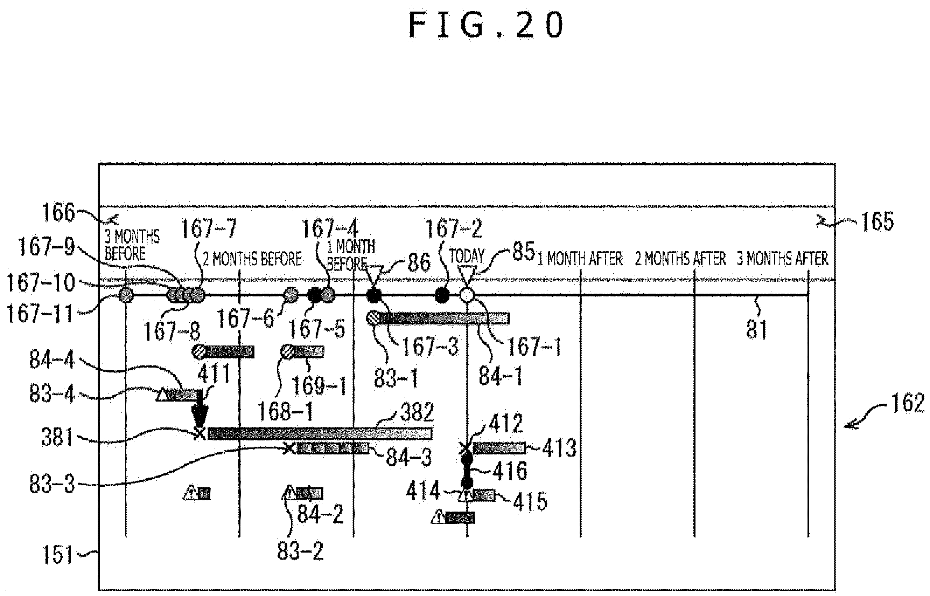

   FIG. 20 is a view depicting an example of a display of a detail display image.
[FIG. 21]

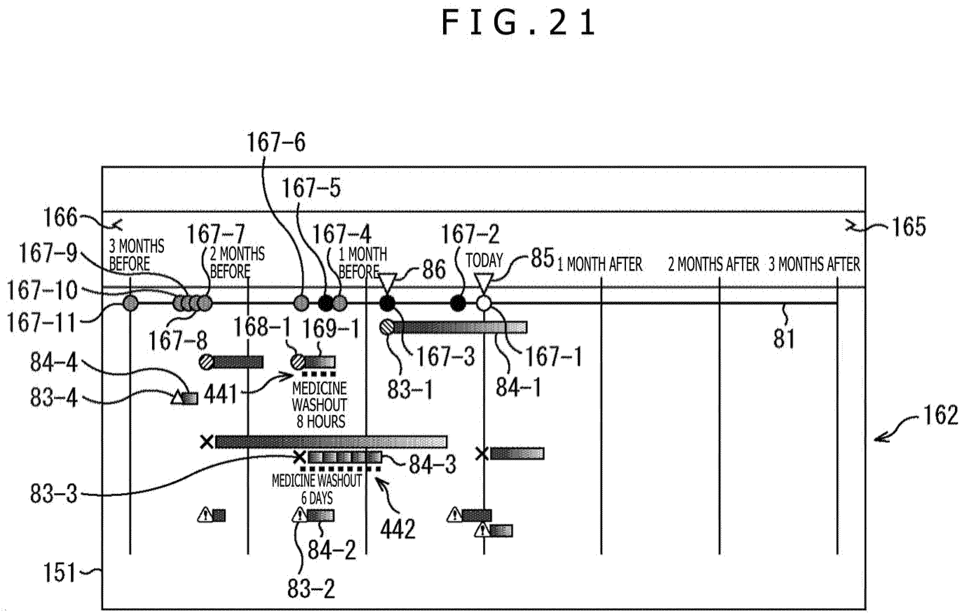

   FIG. 21 is a view depicting an example of another display of the detail display image.
[FIG. 22]

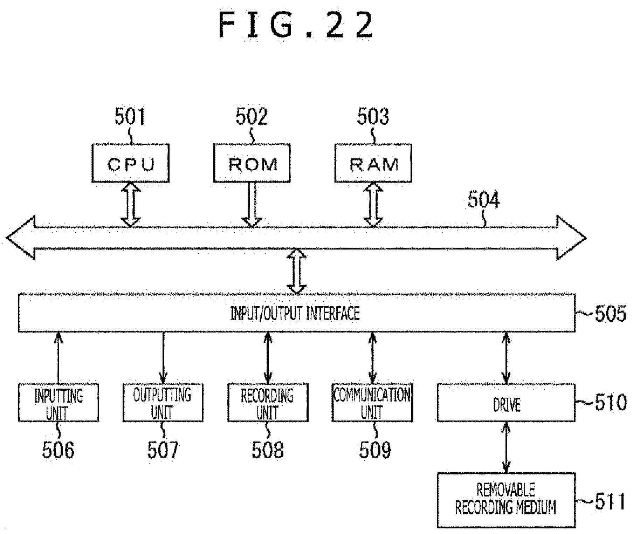

   FIG. 22 is a view depicting an example of a configuration of a computer.

### [Description of Embodiments]

In the following, embodiments to which the present technology is applied are described with reference to the drawings.

### <First Embodiment>

### <Example of Configuration of Information Processing System>

The present technology displays, in a display form according to specific medication history information, different medication history information so as to make it possible for a user, who receives presentation of medication history information such as a relation between medicines, to grasp necessary information more quickly and intuitively.

FIG. 1 is a view depicting an example of a configuration of an embodiment of an information processing system to which the present technology is applied.

This information processing system includes a franchise store system 11, a data center 12, and an IC (Integrated Circuit) card 13 and a portable terminal apparatus 14 that are possessed by a user who is a patient. Further, the franchise store system 11 and the data center 12 are connected to each other through a communication network 15 including a wired or wireless network such as the Internet.

This information processing system can be applied to a case in which various services are provided to users. In the following, in order to make the description concrete, description is given taking a case in which the franchise store system 11 or the portable terminal apparatus 14 uploads medication history of an electronized drug notebook or the like into the data center 12 such that the medication history data is suitably read by the franchise store system 11 or the portable terminal apparatus 14.

Here, the medication history data includes prescription information that is information of a prescription issued, for example, by a doctor and dispensing history information indicative of a history of dispensing performed by a pharmacy on the basis of a prescription. Further, the medication history data includes also an actual taking situation or taking period reported (inputted) by a patient itself or an employee of a medical facility, taking report information indicative of a remaining amount of a medicine, and side effect report information that is information relating to a side effect of a medicine reported (inputted) by a patient itself or an employee of a medical facility, and so forth.

It is to be noted that uploading of medication history data is performed by a service franchise store utilized by the user or by the user itself after authentication utilizing the IC card 13 or in-person authentication as occasion demands.

The franchise store system 11 is provided in a service franchise store that provides a predetermined service. In this example, the franchise store system 11 is provided, for example, in a pharmacy in which a user purchases a prescribed medicine or in a hospital in which a user receives regular outpatient treatment.

The franchise store system 11 has a franchise store terminal apparatus 21 including a computer or the like, a receipt computer 22, and an information identification reader 23. In this example, the franchise store terminal apparatus 21 and the receipt computer 22 are connected to each other by a local network provided in the franchise store system 11, and the information identification reader 23 is connected to the franchise store terminal apparatus 21.

For example, the franchise store terminal apparatus 21 or the receipt computer 22 performs various processes in response to an inputting operation of a pharmacist or the like. In particular, for example, the receipt computer 22 generates medication history data of a user in response to an inputting operation of a pharmacist or the like and records the medication history data. Further, for example, the franchise store terminal apparatus 21 transmits medication history data generated by the receipt computer 22 to the data center 12 through the communication network 15 such that the data center 12 records the medication history data.

Meanwhile, the information identification reader 23 reads out a personal identification ID that is an ID unique to a user or personal information from the IC card 13 of the user and supplies the read out information to the franchise store terminal apparatus 21. The franchise store terminal apparatus 21 utilizes the personal identification ID or personal information supplied thereto from the information identification reader 23 for authentication of the user itself or the like or transmits information or the like required for accessing to the medication history data managed by the data center 12 to the portable terminal apparatus 14 by short-range communication.

It is to be noted that, not only from the IC card 13 but also from the portable terminal apparatus 14 having an IC chip equivalent to that the IC card 13 has or the like, a personal identification ID or personal information may be read out by the information identification reader 23.

Here, although the personal identification ID is one of kinds of personal identification information capable of uniquely specifying a user, it is information with which, even if an unrelated person merely acquires the personal identification ID, generally it is impossible (difficult) to specify the individual user who is identified by the personal identification ID.

Further, a medium that provides an opportunity for acquiring a personal identification ID is not limited to an IC card or the like in which the personal identification ID is recorded directly but may be any unique medium for which rewriting or replacement is impossible. For example, where unique information is acquired as a personal identification ID from living body information, the medium for acquiring the personal identification ID is part or the entirety of a living body.

It is to be noted that the franchise store terminal apparatus 21 need not be installed in a franchise store if it is an apparatus that can acquire a personal identification ID or personal information and can exchange information with the data center 12 or the portable terminal apparatus 14 through a communication network, and may be, for example, a portable terminal apparatus.

The data center 12 manages medication history data supplied thereto from the franchise store system 11 or the portable terminal apparatus 14 and permits reading of the recorded medication history data in response to a request from the franchise store system 11 or the portable terminal apparatus 14.

The data center 12 includes a recording unit 24, a communication unit 25 and a control unit 26.

The recording unit 24 records various data such as medication history data. For example, the recording unit 24 records, for each user, a personal identification ID of the user and medication history data of the user in an associated relation with each other. Meanwhile, the communication unit 25 communicates with the franchise store system 11 or the portable terminal apparatus 14 through a communication network such as the communication network 15, and the control unit 26 updates the medication history data and so forth recorded in the recording unit 24 as occasion demands.

The IC card 13 is an IC card that is used suitably when a user who is to enjoy a service provided in a service franchise store or the like in which the franchise store system 11 is installed enjoys the service. This IC card 13 is issued for each user. The IC card 13 has recorded therein a personal identification ID for authentication of a user whose is a service user and personal information that includes a name, the date of birth, a sex, a sign, a number and so forth of a certificate of insurance and so forth of the user. It is to be noted that the personal information recorded in the IC card 13 may additionally include an address, a telephone number or the like of the user.

The IC card 13 communicates with the information identification reader 23 to transmit the personal identification ID or personal information recorded therein to the information identification reader 23.

The portable terminal apparatus 14 includes a portable telephone set or the like possessed by the user and communicates with the franchise store system 11 or the data center 12 through a communication network not depicted or the communication network 15 to perform exchange of information as occasion demands. For example, the portable terminal apparatus 14 issues a request for information of the user itself on the basis of information recorded therein, receives and displays medication history data from the data center 12, or transmits such medication history data to the data center 12 for updating of the same.

It is to be noted that, although, in the example of FIG. 1, only one franchise store system 11 is depicted in the information processing system, actually the information processing system includes a plurality of franchise store systems 11.

### <Example of Medication History Data>

Here, an example of medication history data recorded in the recording unit 24 of the data center 12 is described. For example, in the recording unit 24, dispensing history information depicted in FIG. 2 or taking report information and side effect report information depicted in FIG. 3 are recorded as the medication history data.

In the example depicted in FIG. 2, a personal identification ID is recorded in an associated relation with dispensing history information including a name of a medicine, the dispensing date, a prescription ID, a number of days of taking, an amount upon one taking, a use method and a number of times of taking per one day.

Here, the "name of medicine" indicates a name of the medicine prescribed on the basis of a prescription for a user who is a patient, and the "dispensing date" indicates a date of dispensing of the medicine. Further, the "prescription ID" is information indicative of a prescription in which the dispensed medicine is prescribed, and the "number of days of taking" indicates the number of days for the prescription of the medicine, namely, the number of days during which the medicine is to be taken.

Further, the "amount upon one taking" indicates an amount to be taken (medication amount) upon taking of the medicine in one taking (once), and the "use method" depicts a taking method of the medicine. For example, "internal use" indicates that the medicine is an internal medicine. Since this use method indicates a taking method, it also is information indicative of a type of the medicine such as an internal medicine or a conscription medicine. Further, the "number of times of taking per one day" indicates how many times the medicine is to be taken a day, and for example, "three times" indicates that the medicine is to be taken three times a day.

According to the dispensing history information, a prescription period of each medicine can be specified from a dispensing date, a number of days of taking, an amount upon one taking, a use method, a number of times of taking per one day and so forth.

Further, in FIG. 3, taking report information and side effect report information as medication history data are depicted, and such taking report information and side effect report information are recorded in an associated relation with a personal identification ID.

For example, the taking report information includes a name of a medicine, the dispensing date, a prescription ID, a number of days of taking, an amount upon one taking, a number of times of taking per one day, a remaining medicine amount and a hearing date.

Here, the "name of medicine," the "dispensing date" and the "prescription ID" are similar to those in the dispensing history information described hereinabove. Meanwhile, the "number of days of taking" in the taking report information indicates the number of days during which the medicine is actually taken by the user, and the "amount upon one taking" indicates an amount taken actually by the user in one taking. The "number of times of taking per one day" indicates how many times the medicine is actually taken a day by the user, and the "remaining medicine amount" is a remaining amount of the medicine, and the "hearing date" is a date on which hearing of the "number of days of taking" or the "remaining medicine amount" is performed.

For example, the taking report information is information obtained by inputting, when hearing is performed for a user in regard to a taking situation of a medicine taken already or being taken (not taken) by a medical employee such as the care staff or the like, a remaining amount of the medicine and so forth, a result of the hearing. Alternatively, the taking report information may be information inputted by the user itself who is a patient, a protector of the user or the like.

In the taking report information, from the dispensing date, number of days of taking and remaining medicine amount, whether the medicine has been taken already or is being taken, the remaining amount of the medicine, an actual taking period of the medicine and so forth can be known.

Further, the side effect report information depicted in FIG. 3 includes a side effect occurrence date, a side effect name, a name of a medicine and a prescription ID.

Here, the "side effect occurrence date" indicates a date on which a side effect occurs, and the "name of side effect" indicates a name of the side effect, namely, the substance of the side effect. Further, the "name of medicine" indicates the medicine with regard to which the side effect is reported, and the "prescription ID" is information indicative of a prescription in which the medicine with regard to which the side effect is reported is prescribed. Such side effect report information as just described is information inputted by the patient itself or an employee of the medical facility.

For example, the taking report information of the side effect report information depicted in FIG. 3 is information inputted by an employee of a medical facility or the user itself on the franchise store terminal apparatus 21, the portable terminal apparatus 14, a web page or the like such that a result of the input is supplied to the data center 12 and recorded into the recording unit 24.

### <Example of Presentation of Medication History Data>

Now, referring to FIG. 4 to FIG. 6, an example is described in which, for example, the franchise store terminal apparatus 21 acquires necessary medication history data from the data center 12 and displays information relating to a medication history of a user who is a patient on the basis of the acquired medication history data.

In such a case as just described, the franchise store terminal apparatus 21 displays, for example, a medication history display image 41 depicted in FIG. 4 as information relating to a medication history.

It is to be noted that, in the following, a case in which a user who is a patient visits a pharmacy that is a franchise store in which the franchise store system 11 is installed and has medicines, which are indicated in a predetermined prescription, dispensed by a pharmacist in the pharmacy is described as an example. In the following, medicines dispensed in the pharmacy at this time are especially referred to as currently dispensed medicines.

Further, the medication history display image 41 depicted in FIG. 4 is displayed on the franchise store terminal apparatus 21 in order to allow a pharmacist or the patient itself to confirm, after the current dispensing, information relating to the dispensed medicines and information relating to medicines dispensed in the past. In this case, the medication history data relating to the current dispensing may have already been uploaded in the data center 12 or may not have been uploaded as yet in the data center 12. Further, although an example in which the medication history display image 41 is displayed after dispensing is described here, the medication history display image 41 may be displayed otherwise before dispensing.

The medication history display image 41 depicted in FIG. 4 is generated on the basis of a personal identification ID or personal information read out from the IC card 13 or the portable terminal apparatus 14 by the information identification reader 23, medication history data acquired from the data center 12 by the franchise store terminal apparatus 21, newly generated medication history data retained by the receipt computer 22 and so forth. In the following, a screen image state in which the medication history display image 41 is displayed is referred to also as medication history display screen image.

On the medication history display image 41, principally a personal information displaying region 51, an attribute information selection region 52, a medication history plot displaying region 53 and a medication history displaying region 54 are provided. Further, on the lower side in the figure in the medication history displaying region 54 of the medication history display image 41, also various buttons such as a button for changing a character size and a button for causing allergy or a case history of the patient itself to be displayed are provided.

Here, FIG. 5 is a view depicting, in an enlarged scale, the portions of the attribute information selection region 52 and the medication history plot displaying region 53 of the medication history display image 41 depicted in FIG. 4, and FIG. 6 is a view depicting, in an enlarged scale, the portion of the medication history displaying region 54 of the medication history display image 41 depicted in FIG. 4. In the following, the medication history display image 41 is described with reference to FIGS. 4 to 6.

In the personal information displaying region 51, information relating to a patient itself such as personal information of the patient read out, for example, from the IC card 13 or the like.

In the present example, the name "Masaaki KURAMOTO" of the patient, the date of birth "December 11, Heisei 3 [1991]" of the patient, a sign and a number "123/123456" of a certificate of insurance and so forth are displayed.

Further, in the attribute information selection region 52 provided at the lower side in FIG. 4 of the personal information displaying region 51, check boxes for selecting attribute information to be displayed in the medication history plot displaying region 53 or the medication history displaying region 54 as a piece of information relating to a medicine and so forth are displayed.

Here, the attribute information is information relating to a medicine belonging to a predetermined attribute, and the attribute information here is information indicative of an attribute of the medicine, namely, information indicative of what medicine the medicine belonging to the attribute is. An attribute of a medicine is determined on the basis of the medicine or on a medicine of a specific noticed dispensing date of the medicine, and one medicine sometimes belongs to a plurality of attributes.

The attribute information selection region 52 for selecting such attribute information to be made a display target as just described can be regarded as a region for selecting, from among a plurality of attributes, an attribute of attribute information of a display target, namely, an attribute of a medicine whose attribute information is to be displayed.

In particular, as depicted in FIG. 5, in the attribute information selection region 52, a side effect mark 61, an under-taking mark 62, a same medicine mark 63, a contraindication-for-coadministration mark 64 and a precaution-for-coadministration mark 65 are displayed as marks (each hereinafter referred to as attribute mark) indicative of attributes of a medicine (attribute information).

Here, the side effect mark 61 is an attribute mark indicative of an attribute to which a medicine whose side effect occurs with a patient as a result of taking belongs, and in the following description, an attribute to which such a medicine as just described belongs is referred to especially also as side effect attribute. Attribute information of a medicine having the side effect attribute is side effect information indicating that, as a result of taking of the medicine, a side effect has occurred with the patient. It is to be noted that the side effect indicated by the side effect information may be any side effect such as, for example, a side effect inputted by a patient, a side effect confirmed through a diagnosis by a doctor, or "drowsiness" included in information of the medicine.

Further, in the example depicted, also characters "side effect" indicating that the side effect mark 61 is an attribute mark representative of the side effect attribute are displayed at the left side in FIG. 5 of the side effect mark 61.

The under-taking mark 62 is an attribute mark representative of an attribute to which a medicine that is being currently taken belongs, and in the following description, an attribute to which such a medicine as just described belongs is referred to especially also as under-taking attribute. The attribute information of a medicine having the under-taking attribute is in-medication information indicating that the medicine is currently in medication. In the present example, also characters "under taking" indicating that the under-taking mark 62 is an attribute mark representative of the under-taking attribute is displayed at the left side in FIG. 5 of the under-taking mark 62.

The same medicine mark 63 is an attribute mark representative of an attribute to which a medicine same as a medicine dispensed for a user (patient) on a specific noticed dispensing date belongs, and in the following description, an attribute to which such a medicine as just described belongs is referred to especially also as same medicine attribute. The attribute information of a medicine of the same medicine attribute is same medicine information indicating that the medicine is same as a medicine dispensed for the user at a noticed specific dispensing date. In this example, also characters "same medicine as that of current dispensing" indicating that the same medicine mark 63 is an attribute mark representative of the same medicine attribute are displayed at the left side in FIG. 5 of the same medicine mark 63.

It is to be noted that the noticed specific dispensing date may be any date if the date is a dispensing date of the medicine prescribed for the user who is a patient such as a dispensing date selected by the user or the like, a dispensing date that becomes a specific reference or the latest dispensing date. However, the following description is given assuming that the date on which current dispensing is performed is the noticed specific dispensing date.

Further, it is assumed here that the medicine same as the medicine dispensed currently includes not only a medicine same as the medicine dispensed currently but also medicines of same components as those of the medicine dispensed currently such as generic medicines.

It is to be noted that the medicine same as the medicine dispensed currently may not include a medicine different from a medicine same as the medicine dispensed currently, namely, a medicine of same components as those of the medicine dispensed currently such as generic medicines. In short, a medicine that is fully same as the medicine dispensed currently and a generic medicine or the like may be distinguished from each other.

The contraindication-for-coadministration mark 64 is an attribute mark representative of an attribute to which a medicine that is contraindicated in coadministration and is included in other dispensed medicines belongs, and in the following description, an attribute to which such a medicine as just described belongs is referred to especially also as contraindication-for-coadministration attribute. The attribute information of a medicine of the contraindication-for-coadministration attribute is contraindication-for-coadministration information indicating that a medicine that is contraindicated in coadministration is included in dispensed medicines. In this example, also characters "contraindication for coadministration" indicating that the contraindication-for-coadministration mark 64 is an attribute mark indicative of the contraindication-for-coadministration attribute are displayed at the left side in FIG. 5 of the contraindication-for-coadministration mark 64.

The precaution-for-coadministration mark 65 is an attribute mark representative of an attribute to which a medicine that is precautioned in coadministration and is included in other dispensed medicines belongs, and in the following description, an attribute to which such a medicine as just described belongs is referred to especially also as precaution-for-coadministration attribute. The attribute information of a medicine of the precaution-for-coadministration attribute is precaution-for-coadministration information indicating that a medicine that is precautioned in coadministration is included in dispensed medicines. In this example, also characters "precaution for coadministration" indicating that the precaution-for-coadministration mark 65 is an attribute mark indicative of the precaution-for-coadministration attribute are displayed at the left side in FIG. 5 of the precaution-for-coadministration mark 65.

It is to be noted that contraindication for coadministration or precaution for coadministration is a stage of a risk of a coadministration (taking coadministration) of medicines between which an interaction occurs. More particularly, the contraindication for coadministration includes relative contraindication for coadministration and absolute contraindication for coadministration, and the magnitudes of the risks at the stage of the risks of the contraindication for coadministration and the precaution for coadministration have such a relation as given below.
"precaution for coadministration" < "relative contraindication for coadministration" < "absolute contraindication for coadministration"

In the following, it is assumed that any of the relative contraindication for coadministration and the absolute contraindication for coadministration is referred to merely as contraindication for coadministration. It is to be noted that, where the relative contraindication for coadministration and the absolute contraindication for coadministration are to be distinguished from each other, a relative coadministration contraindication mark and an absolute coadministration contraindication mark may be used in place of the contraindication-for-coadministration mark 64 such that attributes corresponding to the relative coadministration contraindication mark and the absolute coadministration contraindication mark can be selected separately from each other.

Further, at the left side in FIG. 5 of the attribute marks, a check box 66 to a check box 70 for selecting attribute information of an attribute represented by a corresponding attribute mark as attribute information to be displayed are arranged. A user or the like can operate any of the check boxes to cause a check mark to be displayed in the check box to select the attribute corresponding to the check box, namely, the corresponding piece of attribute information. In the following, an attribute selected so as to be displayed through a check box is referred to especially also as selection attribute.

Further, when one of the attributes is selected, where some other attribute has a strong association with the selection attribute, also the attribute having the strong association may be automatically selected.

In this example, it is determined in advance that the contraindication-for-coadministration attribute is an attribute having a stronger association than the precaution-for-coadministration attribute, and if the precaution-for-coadministration attribute is selected by an operation to the check box 70 by a user or the like, then also the contraindication-for-coadministration attribute is selected at the same time.

It is to be noted that, in the present embodiment, if the precaution-for-coadministration attribute is not selected by a user or the like, even if the contraindication-for-coadministration attribute is selected, then the precaution-for-coadministration attribute is not selected automatically in response to the selection. Further, although an example in which a different attribute having a stronger relation is automatically selected is described here, such automatic selection may not be performed while each attribute is selected by a user.

The franchise store terminal apparatus 21 has recorded therein in advance such information that indicates an attribute of a strong association as related attribute information. It is to be noted that the related attribute information may be acquired from the data center 12 or some other apparatus by the franchise store terminal apparatus 21.

Also a detail button 71 is provided in the attribute information selection region 52. If this detail button 71 is operated, then a detail display image that is an image that displays information displayed in the medication history plot displaying region 53 more in detail. For example, the detail display image is displayed in an overlapping relation on the medication history display image 41. In the following description, a screen image state in which a detail display image is displayed is referred to also as detail display screen image. Accordingly, if the detail button 71 is operated, then the state of the display screen image transits from a medication history displaying screen image to a detail display screen image.

Further, selection of attribute information in the attribute information selection region 52 may be performed every time a medication history display image 41 is displayed or a result of the selection by a user or the like may be recorded as selection attribute information into the franchise store terminal apparatus 21 or the data center 12.

For example, where selection attribute information of a user (patient) is recorded in an associated relation with the personal identification ID of the user in the franchise store terminal apparatus 21, the selection attribute information is read out. Then, on the basis of the selection result of the attribute (attribute information) indicated by the read out selection attribute information, a check mark is displayed in each check box in the attribute information selection region 52 and the display of the medication history plot displaying region 53 and the medication history displaying region 54 is controlled.

In the medication history plot displaying region 53, information relating to medicines dispensed for a user whose personal information is displayed in the personal information displaying region 51 is displayed in a time series.

In particular, in the medication history plot displaying region 53, a time axis 81 centered at "today" that is a date of current dispensing and indicative of dates in the future and dates in the past as viewed from this day is depicted.

In this example, the right end of the time axis 81 in FIG. 5 is a date three months ahead from the date of the current dispensing indicated by "today" and the left end of the time axis 81 in FIG. 5 is a date three months ago from the date of the current dispensing indicated by "today."

It is to be noted that it may be made possible to change the date that is positioned at the center of the time axis 81 in the medication history plot displaying region 53 from "today" that is the date of the current dispensing to the future side or the past side by an operation of the time axis 81, a scroll bar or the like by a user or the like.

In such a case as just described, for example, a user, a pharmacist or the like can move the period on the time axis 81 displayed in the medication history plot displaying region 53 in the leftward or rightward direction in FIG. 5 by directly slidably operating the time axis 81. In other words, the display position of each piece of information plot-displayed along the time axis 81 in a time series can be slid (moved) in the leftward or rightward direction.

Further, while, in this example, the range of time indicated by the time axis 81 is six months centered at the current dispensing date, it is possible for a user, a pharmacist or the like to change the time range by performing such an operation as a pinch-in or pinch-out operation, a tapping operation or the like for a touch panel. In other words, the time display width of the time axis 81 can be changed.

In particular, for example, if an expansion operation by a pinch-out operation or the like is performed, then the time range displayed on the time axis 81 decreases by a predetermined period of time from six months to four months, two months or the like. In other words, the display width of time displayed on the time axis 81 is increased.

In contrast, for example, if a reduction operation by a pinch-in operation or the like is performed in a state in which the time range displayed on the time axis 81 is two months, then the time range displayed on the time axis 81 increases by a predetermined time period from two months to four months, six months or the like. In other words, the display width of time displayed on the time axis 81 is decreased.

It is to be noted that the change of the time display width of the time axis 81 is not limited to an expansion operation or a reduction operation for a touch panel provided in an overlapping relation on the display unit on which the medication history display image 41 is displayed but may be performed by any other operation. For example, the change of the time display width of the time axis 81 may be performed by an operation of a button provided on the medication history display image 41 or may be performed by a drag operation or the like by an inputting device such as a mouse.

On the time axis 81, a dispensing date mark 82-1 to a dispensing date mark 82-11 indicative of dispensing dates of medicines prescribed for the user (patient) are plot-displayed on the time axis 81 at positions indicative of dates at which the prescribed medicines are dispensed. In this example, for example, the dispensing date mark 82-1 is plot-displayed at the position of the current dispensing date.

It is to be noted that, where the dispensing date mark 82-1 to the dispensing date mark 82-11 need not specifically be distinguished from each other, each of them is referred to merely as dispensing date mark 82.

Further, at the position same as that of each dispensing date mark 82 in the direction of the time axis 81, attribute information that relates to medicines dispensed on the dispensing date indicated by the dispensing date mark 82 and indicates the attribute selected by the attribute information selection region 52 is displayed.

Also medication schedule information in the form of a bar representative of a medication period of a medicine dispensed at the position of each piece of attribute information, namely, on the dispensing date indicated by the dispensing date mark 82 from a start point given by the dispensing date, is displayed. Here, at the right side of the attribute information in FIG. 5, medication schedule information of medicines corresponding to the attribute information is displayed.

The length of the medication period indicated by the medication schedule information is a length determined, for example, from within taking report information included in medication history data and indicative of an actual taking period reported by a patient, an employee of a medical facility or the like, a prescription period included in the prescription information, dispensed taking period information included in the dispensing history information and indicative of an accurate taking period of the dispensed medicine and other information. Further, the length of the medication period indicated by the medication schedule information may be calculated, for example, on the basis of the prescription information or dispensing history information included in medication history data.

It is to be noted that, when to determine the length of a medication period indicated by medication schedule information, it may be made selectable which piece of information is to be used from among such taking report information, prescription period, dispensed taking period information and so forth as described above.

Further, the density or shape of a color of the medication schedule information is determined in response to a taking amount of a medicine, a gradually decreasing period, a type of a medicine and so forth. It is to be noted that a determination method of a display form of the medication schedule information is hereinafter described.

It is to be noted that, when attribute information to be displayed does not exist on the dispensing date at which the dispensing date mark 82 is displayed, only the dispensing date mark 82 is displayed at the position of the dispensing date while attribute information or medication schedule information is not displayed. Further, when attribute information to be displayed does not exist on the dispensing date at which the dispensing date mark 82 is displayed, the dispensing date mark 82 and the medication schedule information may be displayed at the position of the dispensing date.

For example, at the position of the dispensing date at which the dispensing date mark 82-3 is displayed, in-medication information 83-1 that is attribute information of a predetermined medicine is displayed, and also medication schedule information 84-1 whose medication period is displayed in the form of a bar (bar-like display) from a start point that is the position of the in-medication information 83-1.

If the user who is a patient sees the in-medication information 83-1 and the medication schedule information 84-1, then the user can rapidly and intuitively grasp that the user has a medicine in medication, until when the medication period of the medicine continues, how much the medicinal effect of the medicine in the body of the patient is expected and so forth. Especially, since the in-medication information 83-1 is a mark same as the under-taking mark 62, the user can simply and intuitively grasp that there is a medicine in medication.

The current dispensing date on which the current dispensing is performed and at which the dispensing date mark 82-1 is displayed, contraindication-for-coadministration information and precaution-for-coadministration information are displayed as attribute information. The attribute information displayed on the current dispensing date indicates that the medicines dispensed currently include a medicine that is contraindicated in coadministration or precautioned in coadministration when it is taken together with medicines dispensed in the past to the user.

It is to be noted that it may be additionally specified whether medicines dispensed currently include a medicine that is contraindicated in coadministration or precautioned in coadministration when it is taken together with the other medicines dispensed currently and display contraindication-for-coadministration information or precaution-for-coadministration information at the current dispensing date in response to a result of the specification.

Further, at the current dispensing date at which the dispensing date mark 82-1 is displayed, the medication schedule information displayed together with the attribute information indicates a medication schedule (medication period) of a medicine that is prescribed already and scheduled to be taken in the future, and medication of the medicine corresponding to this medication schedule information is not started as yet.

In the following, information displayed especially at the position of each dispensing date is referred to also as medication history information. In particular, the medication history information is information including at least the dispensing date mark 82, and where a medicine corresponding to the attribute information exists at the dispensing date indicated by the dispensing date mark 82, information including the dispensing date mark 82, attribute information and medication schedule information is made the medication history information of the dispensing date.

This medication history information is information obtained from prescription information that is included in medication history data managed by the data center 12 and is information of a prescription issued by a doctor, dispensing history information indicative of a history of dispensing performed by a pharmacy on the basis of the prescription and so forth. Further, the medication history information of each dispensing date is information basically in a unit of a prescription. Especially, the medication history information on the current dispensing date is information, for example, relating to medicines that are dispensed already and are scheduled to be medicated (taken) in the future.

The medication history information can be regarded as information relating to medicines obtained from medication history data associated with a personal identification ID. Especially, the medication history information is information itself relating to medicines or a relation between medicines, which is obtained from information itself included in medication history data or information relating to medicines included in the medication history data.

In the medication history plot displaying region 53, medication history information including the dispensing date mark 82 and so forth are plot-displayed at the position of each dispensing date. In other words, pieces of medication history information are displayed in a time series on the time axis 81.

Further, although attribute information of a selected attribute is displayed at the position of the date at which the dispensing date mark 82 is displayed, attribute information of the same attribute sometimes becomes a displaying target in regard to a plurality of medicines dispensed on the dispensing date. In this case, it seems a possible idea to display medicine names and so forth in the medication history plot displaying region 53 and display attribute information and medication schedule information for each medicine.

However, since the displaying region of the medication history plot displaying region 53 is restricted, medication schedule information is displayed on the basis of information relating to a medicine having the highest priority (significance) from among the medicines corresponding to the attribute information of the displaying target.

Here, the medicine having the highest significance is, for example, a medicine whose medication period is longest or a like medicine. If the longest medication period is displayed as the medication schedule information, then a pharmacist, a doctor or the like who sees the medication schedule information can acquire sufficient information in regard to the medication period.

Further, for example, at the position of the dispensing date at which the dispensing date mark 82-6 is displayed, precaution-for-coadministration information 83-2 and contraindication-for-coadministration information 83-3 are displayed as attribute information, and also medication schedule information 84-2 and medication schedule information 84-3 are displayed together with the precaution-for-coadministration information 83-2 and the contraindication-for-coadministration information 83-3.

If the user sees the precaution-for-coadministration information 83-2 and the contraindication-for-coadministration information 83-3, then the user can rapidly and intuitively grasp that the medicines dispensed on the date at which the dispensing date mark 82-6 is displayed include a medicine that is contraindicated in coadministration or precautioned in coadministration when combined with the medicines dispensed currently.

Here, the precaution-for-coadministration information or the contraindication-for-coadministration information displayed at the position of the current dispensing date in the medication history plot displaying region 53 is precaution-for-coadministration information or contraindication-for-coadministration information of a medicine, from among medicines dispensed on the current dispensing date, that is precautioned in coadministration or contraindicated in coadministration when combined with medicines dispensed on other dispensing days.

It is to be noted that, also when medicines dispensed currently include a medicine that is contraindicated in coadministration or precautioned in coadministration when combined with other medicines dispensed currently, contraindication-for-coadministration information or precaution-for-coadministration information may be displayed at the current dispensing date.

In contrast, contraindication-for-coadministration information or precaution-for-coadministration information displayed at a position of a dispensing date other than the current dispensing date in the medication history plot displaying region 53 is contraindication-for-coadministration information or precaution-for-coadministration information, from among the medicines dispensed on the dispensing date, of a medicine that is contraindicated in coadministration or precautioned in coadministration when combined with the medicines dispensed on the current dispensing date.

It is to be noted that, for search for a coadministration of medicines that is contraindicated in coadministration or precautioned in coadministration, a medicine coadministration database which is data indicative of, for example, such a coadministration of medicines as described above is used. For example, such a medicine coadministration database as just described may be recorded anywhere such as the franchise store terminal apparatus 21, data center 12, other servers or the like.

Further, although the dispensing date mark 82 is usually indicated by a round mark "○," the dispensing date mark 82-6 further has a symbol "+" (hereinafter referred to also as additional information mark) added in the round mark. A dispensing date mark 82 in which such an additional information mark as just described is indicated indicates that attribute information and medication schedule information are available which cannot be fully indicated in the region at the date of the dispensing date mark 82 in the medication history plot displaying region 53.

In this case, from among a plurality of pieces of attribute information that become a display target at the position of a date at which the dispensing date mark 82 is indicated, which one of pieces of attribute information is to be displayed preferentially, namely, to be displayed actually, may be selected on the basis of priority degrees (significance degrees) of the pieces of attribute information. In short, attribute information to be displayed actually may be selected in an descending order of the priority degree.

In such a case as described above, for example, attribute priority degree information indicative of a priority degree of each attribute may be recorded in the franchise store terminal apparatus 21. For example, the priority degrees of the attributes indicated by the attribute priority degree information are applied in a descending order of the priority degree to the contraindication-for-coadministration attribute, side effect attribute, in-medication attribute, precaution-for-coadministration attribute, same medicine attribute and so forth.

Accordingly, where a space is available in which only two pieces of attribute information can be displayed, for example, when the contraindication-for-coadministration attribute, precaution-for-coadministration attribute and same medicine attribute correspond to attributes of a displaying target, the contraindication-for-coadministration information and the precaution-for-coadministration information are displayed as attribute information preferentially in accordance with the priority degrees indicated by the attribute priority degree information. Then, the additional information mark is indicated in the dispensing date mark 82 displayed at the dispensing date.

Further, for example, where the additional information mark is applied to the dispensing date mark 82, if a detail display screen image (detail display image) is displayed, then it is possible to confirm the attribute information and the medication schedule information, which are not displayed because the medication history plot displaying region 53 is not sufficient to display them,. For example, the detail display screen image may be displayed not only when the detail button 71 is operated as described above but also when the dispensing date mark 82 in which the additional information mark is displayed is operated.

Further, if a plurality of pieces of attribute information for a same medicine become a displaying target, then both of the pieces of attribute information may be displayed or only the attribute information of an attribute having a higher priority degree may be displayed on the basis of the attribute priority degree information. Alternatively, both of the pieces of attribute information may be time divisionally alternately.

Further, in the medication history plot displaying region 53, side effect information 83-4 is displayed as attribute information, for example, at the position of the dispensing date at which the dispensing date mark 82-10 is displayed, and also medication schedule information 84-4 is displayed together with the side effect information 83-4.

If the user sees the side effect information 83-4 and the medication schedule information 84-4, then the user can rapidly and intuitively grasp that the medicines dispensed on the date at which the dispensing date mark 82-10 is displayed include a medicine whose side effect is reported from the patient or the like.

Further, in the medication history plot displaying region 53, a pointer 85 and another pointer 86 are displayed at an upper side in FIG. 5 of a predetermined dispensing date mark 82.

The pointer 85 and the pointer 86 are pointers for selecting, when detail medication history information that is more detailed information than the medication history information displayed in the medication history plot displaying region 53 is to be displayed in the medication history displaying region 54, of which dispensing date detail medication history information is to be displayed. Especially, the pointer 85 is a pointer for selecting a noticed specific dispensing date described hereinabove, namely, the dispensing date mark 82 of the dispensing date.

In this example, the dispensing date mark 82-1 at the position of the current dispensing date is selected by the pointer 85, and in this example, the pointer 85 is blocked against operation to select the dispensing date mark of any other dispensing date. Further, the dispensing date mark 82-3 is selected by the pointer 86. Therefore, in the medication history displaying region 54, detail medication history information of the dispensing date indicated by the dispensing date mark 82-1 and detail medication history information of the dispensing date indicated by the dispensing date mark 82-3 are displayed.

It is to be noted that, although an example in which a dispensing date is designated by the pointer 85 or the pointer 86 is described here, the dates designated by the pointers are not limited to dispensing dates but may be any date such as a prescription date, a medication date or the like of the medicine.

A user, a pharmacist or the like can select (change) the dispensing date of detail medication history information to be displayed in the medication history displaying region 54 by operating the pointer 86 to move the pointer 86 to the position of a desired dispensing date mark 82. It is to be noted that it may be made possible to select a dispensing date of detail medication history information to be displayed in the medication history displaying region 54 by a user, a pharmacist or the like directly selecting a dispensing date mark 82, attribute information, medication schedule information or the like.

Further, it may be made possible to select a noticed specific dispensing date, in short, a dispensing date of detail medication history information to be displayed in the medication history displaying region 54, by a user, a pharmacist or the like moving the pointer 85 to the position of a desired dispensing date mark 82.

In such a case as just described, also a display of the characters "same medicine as that of current dispensing" that are displayed together with a same medicine mark 63, characters "interaction with current dispensing" displayed together with the contraindication-for-coadministration mark 64 and the precaution-for-coadministration mark 65 and so forth is changed suitably in response to a result of selection of a dispensing date by the pointer 85. For example, the display is changed such that a portion of "current dispensing" in those displays becomes a display of dispensing on the dispensing date indicated by the pointer 85, namely, becomes a display of dispensing at the specific noticed dispensing date. Furthermore, in this case, also with regard to the same medicine information, contraindication-for-coadministration information and precaution-for-coadministration information, a display of the attribute information is performed changing the specific noticed dispensing date from the current dispensing date to the dispensing date selected by the pointer 85.

As described above, in the medication history plot displaying region 53, the medication history information on the other dispensing dates is displayed in a display form according to the medication history data (medication history information) on the noticed specific dispensing date, namely, on the current dispensing date, and a selection result of an attribute (attribute information) of medicines. In other words, the medication history information of the other dispensing dates is displayed in a display form according to the attributes of the medicines on the other dispensing dates, which are determined from the medication history data of the current dispensing date and the medication history data of the other dispensing dates or from the medication history data of the other dispensing dates. Consequently, a user, a pharmacist or the like who sees the medication history display image 41 can more rapidly and intuitively grasp necessary information.

For example, since same medicine information, contraindication-for-coadministration information or precaution-for-coadministration information displayed as one of pieces of medication history information on a dispensing date other than the current dispensing date is displayed according to a relation to the medication history information on the current dispensing date, it can be regarded as information whose display form is changed in response to the medication history information on the current dispensing date.

Since medication history information of a different dispensing date is displayed in a display form according to medication history information of the current dispensing date in this manner, information of other medicines related to medicines dispensed currently or the like can be more rapidly and intuitively grasped in a time series.

Accordingly, for example, a pharmacist or the like who sees the medication history information can rapidly and intuitively grasp, in regard to medicines whose attribute information is displayed as medication history information, a noticeable point from the attribute information. Further, a pharmacist or the like can rapidly and intuitively grasp, in regard to medicines only whose dispensing date mark 82 is displayed as medication history information, that there is no necessity to be noticed specifically.

Further, for example, in a pharmacy or a medical facility, it is possible to more rapidly acquire information necessary for dispensing or to rapidly and accurately grasp information relating to a medication history of a patient that is significantly important for determination of a coadministration or the like upon diagnosis by a doctor. Further, not only an employee of a medical facility but also a patient itself can readily perform determination of a coadministration of medicines upon purchase of a medicine on the market, and improvement in self medication can be anticipated.

In the medication history displaying region 54, a detail medication history information displaying region 91 and another detail medication history information displaying region 92 are provided as displaying regions for detail medication history information as depicted in FIG. 6.

Here, the detail medication history information displaying region 91 is a region in which detail medication history information of a dispensing date selected by the pointer 85, namely, of a dispensing date at which the dispensing date mark 82 selected by the pointer 85 is plotted, is displayed. Similarly, the detail medication history information displaying region 92 is a region in which detail medication history information of a dispensing date selected by the pointer 86 is displayed.

For example, in a field at the upper side in FIG. 6 of the detail medication history information displaying region 91, namely, in a field indicated by an arrow mark A11, information relating to a medical facility by which medicines relating to detail medication history information displayed in the detail medication history information displaying region 91 are prescribed or a pharmacy in which dispensing of the medicines is performed is displayed.

Further, in each of fields provided at the lower side in the figure in a field indicated by the arrow mark A11, information relating to a medicine dispensed on the dispensing date selected by the pointer 85 is displayed for each medicine.

For example, in a field indicated by an arrow mark A12, information of "Clarithromycin tablet 200 mg" indicative of a dispensed medicine, an original medicine name "Clarith tablet 200" of the medicine, a prescription dose "total 14 tablets" of the medicine, a type, a taking method and a prescription period "(internal use, separate 2, after breakfast and dinner, ...) 2 tablets per 1 day, for 14 days" of the medicine and so forth is displayed. It is to be noted that the "internal use" indicates that the type of the medicine is the internal medicine, and "separate 2" indicates the medicine is to be taken separately twice a day. Further, the "2 tablets per 1 day, for 14 days" indicates that the medicine to be taken by two tablets a day is dispensed for 14 days.

Further, in a field indicated by the arrow mark A12, also same medicine information 101-1 and contraindication-for-coadministration information 101-2 that are attribute information of the medicines with regard to which various kinds of information are displayed in the field are displayed, and the field indicated by the arrow mark A12 is displayed in a display form of a color or the like that is determined by attribute information or the like displayed in the field.

The same medicine information 101-1 and the contraindication-for-coadministration information 101-2 are marks of colors or shapes similar to those of the same medicine mark 63 and the contraindication-for-coadministration mark 64 displayed in the attribute information selection region 52, respectively. Further, the numeral "3" displayed in the same medicine information 101-1 indicates that the medicine described in the field in which the same medicine information 101-1 is displayed is a third medicine with regard to which same medicine information is displayed from among the medicines included in the detail medication history information of the current dispensing.

Here, for a medicine with regard to which same medicine information is displayed in the detail medication history information displaying region 91, a same medicine exists in the medicines displayed in the detail medication history information displaying region 92, more particularly, a medicine of same components exists including generic medicines.

Further, for example, in a field indicated by an arrow mark A13, information of "Asverin tablet 30 20 mg" indicative of a dispensed medicine, a prescription dose "total 7 tablets" of the medicine, a type, a taking method and a prescription period "(internal use, separate 1, before breakfast) 1 tablet per 1 day, for 7 days" of the medicine and so forth is displayed.

Further, in the field indicated by the arrow mark A13, also precaution-for-coadministration information 101-3 that is attribute information of medicines with regard to which various types of information are displayed and new medicine information 102 that indicates that the medicine is a new medicine that has not been prescribed as yet for the user (patient) are displayed. Here, whether a medicine is to be displayed in the new medicine information 102 can be specified by comparing medicines included in the prescription information of the prescriptions in the past or dispensing history information included in the medication history data and the medicines dispensed currently with each other.

In the detail medication history information displaying region 91, also a scroll bar 103 for scrolling the display field for detail medication history information displayed in this field is provided. Further, at the lower side in the figure of the detail medication history information displaying region 91, also an interaction confirmation button 104 is provided.

The interaction confirmation button 104 is a button for causing detailed description of contraindication for coadministration or precaution for coadministration of medicines, for which contraindication-for-coadministration information or precaution-for-coadministration information is displayed in the detail medication history information displaying region 91, to be displayed.

For example, if the interaction confirmation button 104 is operated, then an interaction detail display image in which detail information of contraindication for coadministration or precaution for coadministration is displayed in an overlapping relation with the medication history display image 41. At this time, for example, as regards contraindication for coadministration, it is displayed in detail whether it is relative contraindication for coadministration or absolute contraindication for coadministration. In the following description, a screen image state in which an interaction detail display image is displayed is referred to also as interaction detail display screen image.

In particular, for example, in the interaction detail display screen image, such information as medicines with regard to which contraindication-for-coadministration information or precaution-for-coadministration information is displayed from among the medicines dispensed currently, medicines that are prescribed to the user (patient) in the past and that is contraindicated in coadministration or precautioned in coadministration together with the medicines, symptoms (interactions) caused by a coadministration of the medicines and so forth is displayed.

The detail medication history information displaying region 92 is a region in which detail medication history information of a dispensing date selected by the pointer 86 is displayed. For example, in a field at an upper side in FIG. 6 of the detail medication history information displaying region 92, namely, in a field indicated by an arrow mark A14, information relating to a medical facility by which medicines regarding the detail medication history information displayed in the detail medication history information displaying region 92 are prescribed or a pharmacy by which dispensing of the medicines is performed is displayed.

Further, in each of fields at the lower side in FIG. 6 of the field indicated by the arrow mark A14, information relating to a medicine dispensed on a dispensing date selected by the pointer 86 is displayed for each medicine.

For example, in a field indicated by an arrow mark A15, information of "Clarithromycin tablet 200 mg" indicative of a dispensed medicine, an original medicine name "Clarith tablet 200" of the medicine, a remaining amount "remaining 2 tablets" of the medicine, a type, a taking method and a prescription period "(internal use, separate 2, after breakfast and dinner, ...) 2 tablets per 1 day, for 7 days" of the medicine and so forth is displayed.

Here, the remaining amount of the medicine can be determined from prescription information or taking report information included in medication history data and dispensing history information, for example. More particularly, the remaining amount of a medicine can be calculated, for example, from a dispensing date of the medicine, a prescription period of the medicine, a prescription dose of the medicine, a taking method of the medicine and so forth.

Furthermore, in the field indicated by the arrow mark A15, also same medicine information 101-4 and in-medication information 101-5 that are attribute information of a medicine with regard to which various kinds of information are stored in the field are displayed. It is to be noted that, in the same medicine information 101-4, a numeral "3" indicating that the medicine is a third medicine in regard to which same medicine information is displayed third in the detail medication history information displaying region 92 is displayed. From this, it can be rapidly and intuitively grasped that the medicine displayed in the field indicated by the arrow mark A15 and the medicine displayed in the field indicated by the arrow mark A12 are the same "Clarithromycin tablet 200 mg."

It is to be noted that, while, in the detail medication history information displaying region 91 or the detail medication history information displaying region 92, attribute information such as side effect information regarding each medicine of a dispensing date selected by the pointer 85 or the pointer 86 is displayed, the attribute information to be displayed may be attribute information of an attribute selected by the attribute information selection region 52, or attribute information of not only a selection attribute but all attributes may be displayed.

Further, in the detail medication history information displaying region 92, with regard to each of medicines of not only a dispensing date selected by the pointer 86 but also all other dispensing dates, information relating to the medicines and attribute information of a selection attribute or all attributes may be displayed.

Furthermore, only of medicines whose taking period overlaps with that of a medicine dispensed at the dispensing date selected by the pointer 85, namely, only of medicines whose dispensing date is included in the medication periods indicated by the medication schedule information of the medicines, part or the entirety of the attribute information and information relating to the medicines such as the medicine name may be displayed in the detail medication history information displaying region 92.

In this case, regarding a medicine whose medication period overlaps with that of a medicine of the dispensing date selected by the pointer 85 and that is contraindicated in coadministration or precautioned in coadministration when it is taken together with the medicine, even if the contraindication-for-coadministration attribute or the precaution-for-coadministration attribute is not a selection attribute, information regarding the medicine and the contraindication-for-coadministration information or the precaution-for-coadministration information may be displayed in the detail medication history information displaying region 92. Further, thereupon, an alert display may be performed by performing such a display as to display the contraindication-for-coadministration information or the precaution-for-coadministration information itself or a frame in which such information is displayed so as to be standing out, or an alert may be displayed separately from the contraindication-for-coadministration information or the precaution-for-coadministration information.

Further, it may be made possible for a user, a pharmacist or the like to select in regard to what medicine information is to be displayed in the detail medication history information displaying region 92 such as a medicine whose medication period overlaps with that of a medicine of a dispensing date selected by the pointer 85 or to select attribute information to be displayed in the detail medication history information displaying region 92. In such a case as just described, for example, it may be made possible to perform selection of them through an operation of a button displayed on the medication history display image 41 or through an operation for a setting screen image displayed in a popup display, a setting screen image different from the medication history display image 41 or the like.

Further, in the detail medication history information displaying region 92, a scroll bar 105 for scrolling a display field for detail medication history information displayed in this region is provided. Furthermore, also a side effect button 106 is provided at the lower side in the figure of the detail medication history information displaying region 92.

The side effect button 106 is a button for causing, for a medicine whose side effect information is displayed from among the medicines whose detail medication history information is displayed in the detail medication history information displaying region 92, a medicine name, the substance of a side effect reported in regard to the medicine and so forth to be displayed. If the side effect button 106 is operated, then a side effect image in which the substance and so forth of the side effect are displayed is displayed in an overlapping relation with the medication history display image 41. In the following, a screen image state in which a side effect image is displayed is referred to also as side effect displaying screen image.

It is to be noted that, on the side effect displaying screen image, the substance and so forth of all side effects may be displayed on the basis of side effect report information included in the medication history data of a user (patient).

At the upper side in the figure of the detail medication history information displaying region 92, a previous button 107 and a next button 108 for changing the dispensing date of detail medication history information to be displayed in the detail medication history information displaying region 92 to a previous or next dispensing date with respect to the dispensing date selected currently are provided.

For example, if the previous button 107 is operated, then the dispensing date mark 82-4 that precedes by one mark (in the past) in time to the dispensing date mark 82-3 that is currently selected by the pointer 86 is selected, and the pointer 86 moves to the position of the dispensing date mark 82-4. Then, detail medication history information regarding medicines dispensed on the dispensing date indicated by the dispensing date mark 82-4 selected newly by the pointer 86 is displayed in the detail medication history information displaying region 92.

Similarly, if the next button 108 is operated, then the dispensing date mark 82-2 that succeeds by one mark (in the further) in time to the dispensing date mark 82-3 that is currently selected by the pointer 86 is selected, and the pointer 86 moves to the position of the dispensing date mark 82-2. Then, detail medication history information regarding medicines dispensed on the dispensing date indicated by the dispensing date mark 82-2 selected newly by the pointer 86 is displayed in the detail medication history information displaying region 92.

It is to be noted that the example in which the medication history displaying region 54 is divided into the detail medication history information displaying region 91 and the detail medication history information displaying region 92 and detail medication history information of dispensing dates different from each other are displayed in the detail medication history information displaying regions is described with reference to FIG. 6. However, only the detail medication history information of a dispensing date selected by one of the pointer 85 and the pointer 86 may be displayed in the medication history displaying region 54.

### <Display Example of Detail Display Screen Image>

Now, a display example of a detail display image (detail display screen image) to be displayed when the detail button 71 or the like is operated on the medication history display image 41 is described.

If the detail button 71 is operated to issue an instruction to display a detail display image, then, for example, a detail display image 151 depicted in FIG. 7 is displayed in an overlapping relation with the medication history display image 41. It is to be noted that, in FIG. 7, portions corresponding to those in FIG. 5 or FIG. 6 are denoted by the same reference signs, and description of them is omitted suitably.

Further, while an example in which the detail display image 151 is displayed in an overlapping relation with the medication history display image 41 is described here, the detail display image 151 may be displayed as a screen image different from the medication history display image 41, or information to be displayed in detail on the detail display image 151 may be displayed on the medication history display image 41.

In the detail display image 151, an attribute information selection region 161 for selecting an attribute of attribute information to be displayed and a medication history plot displaying region 162 in which detailed medication history information is to be displayed are provided.

In the attribute information selection region 161, a side effect mark 61, an under-taking mark 62, a same medicine mark 63, a contraindication-for-coadministration mark 64 and a precaution-for-coadministration mark 65 are displayed, and at the left side in the figure with respect to the attribute marks, a check box 66 to a check box 70 are provided.

Accordingly, also in a state in which the detail display image 151 is displayed, a user, a pharmacist or the like can change the selection attribute by operating a check box while referring to the displayed attribute mark.

Further, a time axis 81 is displayed in the medication history plot displaying region 162, and also in this example, with reference to the center position of "today" that is the date of the current dispensing, the right side with respect to the center of the time axis 81 represents dates in the future and the left side with respect to the center of the time axis 81 represents dates in the past similarly as in the case of FIG. 5. Further, the right end in the figure of the time axis 81 is a date three months ahead from the date of the current dispensing indicated by "today," and the left end in the figure of the time axis 81 is a date three months ago to the date of the current dispensing indicated by "today."

In this example, the time range of the attribute information selection region 161 can be changed by operating an enlargement button 163 or a reduction button 164 provided at the upper side in the figure of the attribute information selection region 161. In other words, the time display width of the time axis 81 can be changed.

Further, a user, a pharmacist or the like can move the period on the time axis 81 displayed in the medication history plot displaying region 162 in the leftward or rightward direction in the figure by slidably operating the time axis 81 directly or by operating a slide button 165 or another slide button 166.

For example, if attention is paid to "today" that is the current dispensing date on the time axis 81, then when the slide button 165 is operated, the display position of "today" that is the current dispensing date moves leftwardly in the figure in response to the operation amount of the slide button 165. On the contrary, if the slide button 166 is operated, then the display position of "today" that is the current dispensing date is moved in the rightward direction in the figure in response to the operation amount of the slide button 166.

Furthermore, on the time axis 81 in the medication history plot displaying region 162, a dispensing date mark 167-1 to a dispensing date mark 167-11 corresponding to the dispensing date mark 82-1 to the dispensing date mark 82-11 depicted in FIG. 5, respectively, are plot-displayed in a time series.

It is to be noted that, where there is no necessity to specifically distinguish the dispensing date mark 167-1 to dispensing date mark 167-11 from each other, each of them is referred to simply as dispensing date mark 167 as well.

The dispensing date mark 167 displayed in the medication history plot displaying region 162 is displayed in a display form according to a remaining medicine situation of a medicine dispensed on a dispensing date indicated by the dispensing date mark 167. In other words, also a remaining medicine situation is displayed on the dispensing date mark 167.

For example, in the present example, a legend indicating in what display form the dispensing date mark 167 is to be displayed in response to a remaining medicine situation on each dispensing date is displayed at a portion in the attribute information selection region 161 indicated by an arrow mark A21.

In particular, it is indicated in what manner the dispensing date mark 167 is displayed in each remaining medicine situation of a case in which there is no remaining medicine indicated by characters "no remaining medicine," another case in which there is a remaining medicine indicated by characters "remaining medicine present" and any other case indicated by characters "any other case." Here, the any other case given above signifies a case in which the medication history data does not include information indicative of a remaining medicine situation, another case in which the remaining amount of the medicine cannot be calculated, a further case in which dispensing of the medicine is not completed as yet or a like case.

For example, in the example of FIG. 7, the dispensing date mark 167-1 is displayed in a display form determined for the remaining medicine situation indicated by the characters "any other case."

Further, the dispensing date mark 167-2, dispensing date mark 167-3 and dispensing date mark 167-5 are displayed in a display form described for a remaining medicine situation indicated by the characters "remaining medicine present." The, the dispensing date mark 167-4 and the dispensing date mark 167-6 to the dispensing date mark 167-11 are displayed in a display form determined for a remaining medicine situation indicated by the characters "no remaining medicine."

Accordingly, a user, a pharmacist or the like can rapidly and intuitively grasp the remaining medicine situation of the medicines dispensed on each dispensing date by seeing the dispensing date mark 167.

It is to be noted that, if a plurality of medicines are dispensed on a dispensing date indicated by each dispensing date mark, then the dispensing date marks 167 may be displayed in a display form according to remaining medicine situations of medicines having higher priority degrees. Here, a medicine having a high priority degree is, for example, a medicine having some residual or a like medicine.

Further, since all attribute information relating to medicines on dispensing dates is displayed in the medication history plot displaying region 162, different from a display when the medication history display image 41 is displayed, no additional information mark is displayed in the dispensing date mark 167.

Further, in the medication history plot displaying region 162, at positions same as those of the dispensing date marks 167 in the direction of the time axis 81, all attribute information of a selection attribute relating to medicines dispensed on dispensing dates indicated by the dispensing date marks 167 is displayed. Further, from a start point given by the position of each piece of attribute information, namely, by a dispensing date, also medication schedule information of the medicines dispensed on the dispensing dates indicated by the dispensing date marks 167 and corresponding to the attribute information is displayed.

In this example, for example, at the position of the dispensing date at which the dispensing date mark 167-3 is displayed, the in-medication information 83-1 is displayed as attribute information, and also the medication schedule information 84-1 is displayed together with the in-medication information 83-1. The display of the in-medication information 83-1 and the medication schedule information 84-1 displayed together with the dispensing date mark 167-3 is same as that in the case of the dispensing date mark 82-3 in the medication history display image 41.

In this manner, where the additional information mark is not displayed in the dispensing date mark 82, the attribute information and the medication schedule information displayed for the dispensing date mark 167 corresponding to the dispensing date mark 82 are same as the attribute information and the medication schedule information displayed corresponding to the dispensing date mark 82, respectively.

In contrast, for example, as regards the dispensing date mark 167-6 corresponding to the dispensing date mark 82-6 to which the additional information mark is displayed in the medication history display image 41, same medicine information 168-1 and medication schedule information 169-1 that are not displayed fully by the dispensing date mark 82-6 are displayed further.

In particular, at the position of the dispensing date at which the dispensing date mark 167-6 is displayed in the time direction indicated by the time axis 81, the same medicine information 168-1, contraindication-for-coadministration information 83-3 and precaution-for-coadministration information 83-2 are displayed. Also the medication schedule information 169-1, medication schedule information 84-3 and medication schedule information 84-2 are displayed together with the same medicine information 168-1, contraindication-for-coadministration information 83-3 and precaution-for-coadministration information 83-2.

In this manner, the attribute information and the medication schedule information displayed in the medication history plot displaying region 162 are basically same as the attribute information and the medication schedule information displayed in the medication history plot displaying region 53, respectively. The display of the medication history plot displaying region 162 is different from the display of the medication history plot displaying region 53 in that also the attribute information and the medication schedule information that are not fully displayed in the medication history plot displaying region 53 are displayed. This is because, in comparison with the medication history plot displaying region 53, a sufficient display space is secured in the medication history plot displaying region 162. Further, in the medication history plot displaying region 162, pieces of attribute information and medication schedule information are displayed such that they do not overlap with other and can be seen easily.

It is to be noted that, if a sufficient space can be secured for the medication history plot displaying region 53, then a display similar to that in the medication history plot displaying region 162 may be performed in the medication history plot displaying region 53.

Since a sufficient display space is secured in the medication history plot displaying region 162, for individual pieces of attribute information displayed in the medication history plot displaying region 162, medicines corresponding to the attribute information, namely, names of medicines corresponding to the substance of the information indicated by the attribute information, may be displayed together with the pieces of attribute information.

Further, a taking situation of a medicine taken already or being taken (not completed) may be displayed together with attribute information or a medicine name, or where a medicine is being taken (not completed), the remaining amount of the medicine may be displayed. Alternatively, a dose, a taking method and so forth of the medicine may be displayed together with attribute information or a medicine name.

In this case, for example, from the medication schedule information, a period during which the medicine has been taken and another period during which the medicine is not taken as yet may be displayed in different display forms from each other.

Further, information regarding a medicine such as, for example, a medicine name, a taking situation, a remaining amount of the medicine, a dose, a taking method and so forth may be popup displayed or displayed in a different region when attribute information or medication schedule information is placed into a selection state by a pointer or the like.

Further, if a plurality of medicines correspond to a piece of attribute information displayed on each dispensing date, then attribute information and medication schedule information may be displayed for each of the plurality of medicines. In this case, if a medicine name is displayed together with attribute information, then a user, a pharmacist or the like can acquire more detailed information in regard to each piece of attribute information.

Further, a button 170 is provided in the medication history plot displaying region 162, and if this button 170 is operated, then the detail display image 151 is closed and a state in which only the medication history display image 41 is displayed is entered.

Further, if attribute information, medication schedule information or a dispensing date mark 167 displayed in the medication history plot displaying region 162 is selected, then the selected attribute information or medication schedule information or detail medication history information of the dispensing date corresponding to the dispensing date mark 167 may be displayed.

In this case, for example, the detail display image 151 is closed and only the medication history display image 41 is displayed, and in the detail medication history information displaying region 92 of the medication history display image 41, the attribute information or medication schedule information selected in the detail display image 151 or detail medication history information of the dispensing date corresponding to the dispensing date mark 167 are displayed.

Further, while the foregoing description relates to a case in which the detail display image 151 is displayed in an overlapping relation on the medication history display image 41, for example, if a sufficient displaying region can be secured, then information to be displayed by the medication history display image 41 and the detail display image 151 may be displayed in one screen image. In such a case as just described, for example, the personal information displaying region 51, attribute information selection region 161 and medication history plot displaying region 162 described hereinabove may be displayed at the left side in the display screen image while the medication history displaying region 54 is displayed at the right side in the display screen image.

### <Medication Schedule Information>

Now, medication schedule information displayed together with attribute information in the medication history display image 41 or the detail display image 151 is described.

For example, a case is considered in which medication schedule information of Predonine (registered trademark) that is a medicine prescribed for a user who is a patient is displayed together with attribute information.

At this time, it is assumed that Predonine is prescribed in a medication schedule indicated, for example, by an arrow mark Q11 in FIG. 8. It is to be noted that, in FIG. 8, the horizontal direction indicates time. Further, in the figure indicated by the arrow mark Q11, one square represents Predonine for one tablet, and numerical values represent dates.

Accordingly, in this example, the user who is a patient will takes three tablets of Predonine daily during three days from March 1 to March 3; will take two tablets of Predonine daily during three days from March 4 to March 6; and will take one tablet of Predonine daily for three days from March 7 to March 9.

In such a case as described above, in the medication history display image 41 or the detail display image 151, for example, medication schedule information 201 indicated by an arrow mark Q12 is displayed together with attribute information relating to Predonine.

In this example, the length in the horizontal direction in the figure of the medication schedule information 201 indicates a length of the medication period of Predonine, and the density of a color at each position in a rectangle representative of the medication schedule information 201 indicates a degree of the medicinal effect (duration) of Predonine on the date indicated by the position, namely, a gradual decrease of the medicinal effect. Especially, in the medication schedule information 201, it is indicated that, as the density of a color increases, the degree of the medicinal effect increases.

Further, since the degree of the medicinal effect varies in proportion also to the dose (medication amount) of the medicine or the residual amount in the body of the patient, it can be regarded that the density of the color of the medication schedule information 201 indicates the residual amount or the dose of Predonine in the patient body.

Accordingly, in the present example, a user, a pharmacist or the like who sees the medication schedule information 201 can rapidly and intuitively grasp that the medicine corresponding to the medication schedule information 201 is a medicine whose dose gradually decreases. Such medication schedule information 201 as just described is effective especially, for example, when a pharmacist or the like explains to a patient about the medicine such as a taking method or the like of the medicine.

Further, when Predonine is prescribed in the medication schedule indicated by the arrow mark Q11, in the figure of the medication schedule information in which the horizontal direction is set to the time direction and the vertical direction is set to the degree of the medicinal effect, medication schedule information 202 indicated by an arrow mark Q13 may be displayed together with the attribute information.

In this example, it is indicated that, as the height in the vertical direction in the medication schedule information 202 increases, the degree of the medicinal effect on the date increases, namely, the dose increases. Accordingly, also in the case of the medication schedule information 202, a user, a pharmacist or the like can rapidly and intuitively grasp that the medicine corresponding to the medication schedule information 202 is a medicine whose dose gradually decreases.

It is to be noted that a method for calculating a probable number of medication days by arithmetic operation using a dispensing date, a number of prescription days of a medicine included in medication history data is disclosed in detail, for example, in JP 2014-146251A and so forth.

Meanwhile, medicines include some medicine whose effect by medication continues for several days like Bonalon (registered trademark) (such medicine is hereinafter referred to also as effect lasting medicine).

It is assumed, for example, that four tablets of Bonalon are prescribed at a time, and Bonalon is to be taken once every seven days. In such a case as just described, in the medication history display image 41 or the detail display image 151, medication schedule information 203 indicated by an arrow mark Q14 is displayed, for example, together with attribute information relating to Bonalon.

In this example, the length in the horizontal direction in the figure of the medication schedule information 203 indicates the length of the medication period of Bonalon, and in this example, the length of the medication period is four weeks, namely, 28 days.

Meanwhile, the length of a color at each position in a rectangle representative of the medication schedule information 203 indicates the degree of the medicinal effect (duration) of Bonalon on the date indicated by the position. Especially, in the medication schedule information 203, it is indicated that, as the density of a color increases, the degree of the medicinal effect increases.

Accordingly, a user, a pharmacist or the like who sees the medication schedule information 203 can rapidly and intuitively grasp that the medicine corresponding to the medication schedule information 203 is a medicine whose medicinal effect gradually decreases in one week.

Further, different from the medication schedule information 203 indicated by the arrow mark Q14, the medication schedule information may be displayed taking the vertical direction as the degree of the medicinal effect as in the example indicated by the arrow mark Q13.

In such a case as just described, when four tablets of Bonalon are prescribed at a time and taking of Bonalon is once seven days, for example, medication schedule information 204 indicated by an arrow mark Q15 is displayed together with attribute information.

This example indicates that, as the height in the vertical direction of the medication schedule information 204 increases, the degree of the medicinal effect on the date increases. Accordingly, a user, a pharmacist or the like who sees the medication schedule information 204 can rapidly and intuitively grasp that the medicinal effect of the medicine corresponding to the medication schedule information 204 gradually decreases during one week.

It is to be noted that a method for specifying, from a prescription date, a number of prescription days of a medicine and so forth on the same dispensing date (prescription date) included in medication history data, whether or not each medicine is an effect lasting medicine by an arithmetic operation to estimate a medication schedule of the effect lasting medicine is disclosed in detail, for example, in PCT Patent Publication No. WO 2014/21114 and so forth.

Further, for example, medicines include some medicine called conscription medicine (medicine for toxicity) that is not taken at fixed time but is taken in response to a symptom. For such a conscription medicine, a medication period is not specified in a prescription or the like as in the case of an internal medicine. On the other hand, in order for a conscription medicine to demonstrate a strong effect by taking once, it involves many attention points about contraindication for coadministration.

Therefore, from a prescription in which a conscription medicine is prescribed, medication schedule information of a conscription medicine may be generated with reference to an internal medicine whose number of medication days is longest from among internal medicines prescribed together with the conscription medicine in the prescription.

In particular, it is assumed, for example, that Claritin tablet 10 mg that is an internal medicine is prescribed for seven days for taking three times a day on March 1 and Epipen (registered trademark) injection solution 0.3 mg that is a conscription medicine is prescribed on March 1 simultaneously with the same prescription. In such a case as just described, in the medication history display image 41 or the detail display image 151, for example, medication schedule information 221 and different medication schedule information 222 depicted in FIG. 9 are displayed. It is to be noted that the horizontal direction in FIG. 9 indicates time.

In the example of FIG. 9, the medication schedule information 221 indicates medication schedule information displayed together with attribute information of the Claritin tablet 10 mg that is an internal medicine. The length of this medication schedule information 221 is a length corresponding to seven days that are a medication period of the Claritin tablet 10 mg.

In contrast, the medication schedule information 222 indicates medication schedule information displayed together with attribute information of the Epipen injection solution 0.3 mg that is a conscription medicine. The length of this medication schedule information 222 is equal to the length of the medication schedule information 221. This is because the possibility is high that the medicines prescribed on the same prescribing date, namely, the medicines having the same dispensing date, may be same in medication period.

Further, the medication schedule information 222 that is a conscription medicine is displayed in a display form different from a display form of the ordinary medication schedule information 221 or the like such that it can be distinguished from medication schedule information of medicines of the other types, in particular, from the medication schedule information 221 and so forth.

In this instance, the medication schedule information 222 of a conscription medicine is in a form in which it simulates "T" of "Tonpuku," and a user, a pharmacist or the like who sees the medication schedule information 222 can rapidly and intuitively grasp that the medicine corresponding to the medication schedule information 222 is a conscription medicine. Alternatively, the medication schedule information of a conscription medicine may be displayed by a dotted line or a broken line or may be displayed in a different color.

It is to be noted that an example is described here in which the length of medication schedule information of a conscription medicine is set equal to the length of a medication period that is longest among other internal medicines prescribed in the same prescription and the display form of medication schedule information of a conscription medicine is made a display form different from that of medication schedule information of medicines of the other types. However, also with regard to medicines for external application, for example, the length of the medication schedule information of a medicine for external application may be set equal to the length of the longest medication period among the other internal medicines prescribed in the same prescription, and the display form of the medication schedule information of a medicine for external application may be set to a different form from that of the medication schedule information of the medicines of the other types.

Further, a method for estimating a probable medication period of a conscription medicine or a medicine for external application from a number of prescription days of other medicines or statistical information is disclosed in detail, for example, in PCT Patent Publication No. WO 2014/119401 and so forth.

### <Configuration Example of Franchise Store Terminal Apparatus>

Now, a particular configuration example of the franchise store terminal apparatus 21 that generates and displays the medication history display image 41 described hereinabove is described.

FIG. 10 is a view depicting a configuration example of the franchise store terminal apparatus 21. It is to be noted that, while the franchise store terminal apparatus 21 in FIG. 10 is configured as a single apparatus, the franchise store terminal apparatus 21 may otherwise include a plurality of apparatus.

The franchise store terminal apparatus 21 includes a communication unit 301, an inputting unit 302, a display unit 303, a control unit 304, and a recording unit 305.

Further, an information identification reader 23 is connected to the control unit 304 of the franchise store terminal apparatus 21. The information identification reader 23 communicates with the IC card 13 through non-contact communication, and reads out a personal identification ID and personal information from the IC card 13 and supplies them to the control unit 304.

The communication unit 301 communicates with various apparatus such as the data center 12 and so forth through the communication network 15, and receives and supplies information transmitted thereto to the control unit 304 or transmits information supplied thereto from the control unit 304.

The inputting unit 302, for example, includes a touch panel placed on the display unit 303, a mouse and so forth and supplies a signal according to an operation thereof by a pharmacist or the like to the control unit 304. The display unit 303 includes a liquid crystal display panel or the like and displays an image or the like on the basis of data supplied thereto from the control unit 304.

The control unit 304 controls operation of the entire franchise store terminal apparatus 21. The control unit 304 includes an attribute information generation unit 311, a medication schedule information generation unit 312, a plot position specification unit 313 and a display controlling unit 314.

The attribute information generation unit 311 generates attribute information of a selection attribute on the basis of medication history data. The medication schedule information generation unit 312 generates medication schedule information on the basis of medication history data.

Further, the plot position specification unit 313 specifies a plot position of a dispensing date mark on the time axis 81 and a display form of the dispensing date mark on the basis of the medication history data. The display controlling unit 314 controls display of an image by the display unit 303.

The recording unit 305 records various data such as a personal identification ID and medication history data of a user, relation attribute information, selection attribute information, the medicine coadministration database, attribute priority degree information and so forth and supplies the data to the control unit 304 as occasion demands.

### <Description of Medication History Data Acquisition Process>

Now, operation of the franchise store system 11 is described.

For example, a user who is a patient will go to a pharmacy, which is a service franchise store in which the franchise store system 11 is installed, holding a prescription as occasion demands and request a pharmacist to dispense prescribed medicines. In this case, for example, the pharmacist or the like will ask the user to present the IC card 13 possessed by the user.

Then, if the user holds the IC card 13 over the information identification reader 23 of the franchise store system 11, then the franchise store system 11 acquires medication history data from the data center 12 and starts a medication history data acquisition process for generating medication history data relating to a medicine to be dispensed newly in response to an inputting operation by a pharmacist or the like. In the following, the medication history data acquisition process performed by the franchise store system 11 is described with reference to a flow chart of FIG. 11.

When the user holds the IC card 13 over the information identification reader 23 of the franchise store system 11, at step S11, the information identification reader 23 performs non-contact communication with the IC card 13 to acquire a personal identification ID and personal information from the IC card 13 and supplies them to the control unit 304 of the franchise store terminal apparatus 21. Further, the display controlling unit 314 of the control unit 304 supplies the personal identification ID and the personal information supplied thereto from the information identification reader 23 to the display unit 303 as occasion demands so as to be displayed on the display unit 303. The pharmacist or the like will suitably perform identity verification of the user utilizing the displayed personal information and so forth.

It is to be noted that, although an example in which a personal identification ID and personal information are acquired from the IC card 13 of a user is described here, the personal identification ID and the personal information may otherwise be acquired from the portable terminal apparatus 14 of the user.

After the identify verification of the user is performed, the control unit 304 supplies the acquired personal identification ID and a transmission request for requesting transmission of medication history data associated with the personal identification ID to the communication unit 301.

At step S12, the communication unit 301 transmits the personal identification ID and the transmission request supplied thereto from the control unit 304 to the data center 12 through the communication network 15.

When the data center 12 receives the personal identification ID and the transmission request from the franchise store terminal apparatus 21, it transmits, in response to the transmission request, medication history data recorded in an associated relation with the received personal identification ID to the franchise store terminal apparatus 21 through the communication network 15. It is to be noted that, at this time, necessary information may be exchanged between the data center 12 and the franchise store terminal apparatus 21 to perform authentication of the user.

At step S13, the communication unit 301 of the franchise store terminal apparatus 21 receives the medication history data transmitted thereto from the data center 12 and supplies the medication history data to the control unit 304. Further, the control unit 304 controls the communication unit 301 as occasion demands to supply the received medication history data to the receipt computer 22.

When the medication history data of the user is received in this manner, the pharmacist or the like will operate the receipt computer 22 to cause the medication history data to be displayed as occasion demands and input information relating to medicines of the user to be dispensed currently.

Consequently, at step S14, the receipt computer 22 generates new medication history data relating to the medicines to be dispensed currently on the basis of information inputted in response to an operation of the pharmacist or the like and supplies the medication history data to the franchise store terminal apparatus 21. In particular, the communication unit 301 supplies the medication history data supplied thereto from the receipt computer 22 to the control unit 304. It is to be noted that the new medication history data may be generated before dispensing of medicines or may be generated after medicines are actually dispensed.

Alternatively, the new medication history data may be generated by the franchise store terminal apparatus 21. In such a case as just described, the control unit 304 controls the display unit 303 to display the acquired medication history data as occasion demands and generates new medication history data on the basis of information supplied thereto from the inputting unit 302 in response to an operation of the pharmacist or the like. Further, the new medication history data may not be generated by the franchise store system 11, but new medication history data transmitted from a different apparatus such as a different franchise store system installed in a hospital or the like may be received (acquired) and used by the franchise store terminal apparatus 21.

After the new medication history data is generated in this manner, the medication history data acquisition process is ended.

It is to be noted that the newly generated medication history data is transmitted as data for addition to medication history data recorded in an associated relation with the personal identification ID in the data center 12 together with the personal identification ID to the data center 12 at an appropriate timing and recorded by the data center 12. In particular, the new medication history data is added to the medication history data till then to update the medication history data.

The franchise store system 11 acquires a personal identification ID of a user and acquires medication history data of the user from the data center 12 using the personal identification ID in such a manner as described above. By acquiring the medication history data of a user in this manner, the franchise store system 11 can generate a medication history display image 41.

### <Description of Medication History Displaying Process>

Further, if medication history data of a user is acquired and the inputting unit 302 of the franchise store terminal apparatus 21 is operated to input an instruction to display a medication history display image 41, then the franchise store system 11 performs a medication history displaying process of generating and displaying a medication history display image 41 on the basis of the medication history data.

The medication history displaying process by the franchise store system 11 is described below with reference to a flow chart of FIG. 12. It is to be noted that, at the point of time at which this process is started, medication history data of a user has been acquired from the data center 12 by the medication history data acquisition process described hereinabove with reference to FIG. 11.

At step S41, the control unit 304 generates display data for the personal information displaying region 51 on the basis of the personal information acquired from the IC card 13 by the process at step S11 of FIG. 11.

For example, the control unit 304 generates, from a name or a birthday, a sign, a number and so forth of a certificate of insurance and so forth of the user as the personal information, display data with which the information of them is to be displayed as display data for the personal information displaying region 51.

At step S42, the control unit 304 specifies a selection attribute.

For example, if selection attribute information indicative of a selection attribute selected by a user or the like in the past is recorded in the recording unit 305, then the control unit 304 reads out the selection attribute information from the recording unit 305 and determines the selection attribute indicated by the selection attribute information as specified selection attribute. It is to be noted that the selection attribute information may be acquired from the data center 12 or a different apparatus.

Further, in a state in which, for example, the medication history display image 41 is displayed partly or entirely, a user or the like sometimes operates the inputting unit 302 to cause a check mark to be displayed in a check box in the attribute information selection region 52 to perform a selection operation of a selection attribute.

In such a case, the control unit 304 specifies the selection attribute on the basis of the information supplied from the inputting unit 302 in response to an operation by the user or the like. In other words, an attribute is selected. Further, at this time, the selection attribute information is updated as occasion demands.

Further, at this time, the control unit 304 suitably refers to related attribute information that is stored in the recording unit 305 and indicates an attribute having a strong relation to select an attribute. In particular, when the precaution-for-coadministration attribute is selected as a selection attribute by an operation of the user or the like as described hereinabove, for example, the control unit 304 selects also the attribute of contraindication for coadministration as a selection attribute.

It is to be noted that, although the associated attribute information may be recorded in the recording unit 305, it may be acquired from a different apparatus such as the data center 12.

At step S43, the control unit 304 generates display data for the attribute information selection region 52 on the basis of a result of the specification of a selection attribute.

In particular, the control unit 304 generates display data for the attribute information selection region 52 on the basis of a specification result of a selection attribute such that a check mark may be displayed in the check box of the selection attribute from among the check boxes provided for the individual attributes.

At step S44, the attribute information generation unit 311 generates attribute information relating to the medicines on the basis of the specification result of a selection attribute, the medication history data acquired by the process at step S13 of FIG. 11 and the medication history data obtained by the process at step S14 of FIG. 11.

For example, the attribute information generation unit 311 determines a dispensing date (today) of medicines corresponding to the medication history data obtained by the process at step S14 of FIG. 11, as a noticed specific dispensing date, namely, a dispensing date (current dispensing date) to be indicated by the pointer 85.

Further, the attribute information generation unit 311 refers to the medication history data to extract, from among dispensing dates of the medicines included in the medication history data, dispensing dates of the medicines included in a time range indicated by the time axis 81 including the current dispensing date and successively selects the extracted dispensing date as a dispensing date of a processing target.

The attribute information generation unit 311 generates attribute information of the individual selection attributes on the basis of information relating to the medicines on the dispensing date of the processing target included in the medication history data and information relating to the medicines of the current dispensing date.

In particular, it is assumed that, for example, the side effect attribute, under-taking attribute, same medicine attribute, contraindication-for-coadministration attribute and precaution-for-coadministration attribute are selected as selection attributes.

In this case, the attribute information generation unit 311 specifies, from medicine names of the medicines dispensed on the dispensing date of the processing garget and side effect report information included in the medication history data, whether or not the medicines dispensed on the dispensing date of the processing target include some medicine whose side effect has been reported. Then, if a medicine whose side effect has been reported is found, then the attribute information generation unit 311 generates side effect information by which a mark similar to the side effect mark is displayed for the medicine.

Further, the attribute information generation unit 311 specifies whether or not the medicines dispensed on the dispensing date of the processing target include some medicine that is in medication on the basis of the prescription date, prescription period and prescription amounts of the medicines of the dispensing date of the processing target as well as the taking report information indicative of the actual taking period reported and so forth. Then, if a medicine in medication exists, then the attribute information generation unit 311 generates in-medication information for displaying a mark similar to the in-medication mark in regard to the medicine.

The attribute information generation unit 311 specifies whether there exists a same medicine on the basis of information indicative of medicine names of the medicines of the dispensing date of the processing time. Here, if the dispensing date of the processing target is, for example, the current dispensing date, then it is specified whether or not the medicines dispensed on the current dispensing date include a medicine same as a medicine dispensed on any dispensing date in the past. Further, if the dispensing date of the processing target is any other than the current dispensing date, then it is specified whether the medicines dispensed on the dispensing date include a medicine same as a medicine dispensed on the current dispensing date.

Then, if it is specified that a same medicine is included, then the attribute information generation unit 311 generates same medicine information by which a mark similar to the same medicine mark is displayed for the medicine.

Further, the attribute information generation unit 311 refers to the medicine coadministration database recorded in the recording unit 305 to specify whether the medicines on the dispensing date of the processing target include some medicine that is contraindicated in coadministration or precautioned in coadministration when it is taken together with the medicines dispensed on the current dispensing date. Then, if a medicine that is contraindicated in coadministration or precautioned in coadministration is found, then the attribute information generation unit 311 generates contraindication-for-coadministration information or precaution-for-coadministration information by which a mark similar to a coadministration contraindication mark or a coadministration precaution mark is to be displayed for the medicine.

On the other hand, if the current dispensing date is the dispensing date of the processing target, then the attribute information generation unit 311 specifies whether or not the medicines on the current dispensing date include a medicine whose coadministration with a medicine dispensed in the past becomes contraindication for coadministration or precaution for coadministration, and generates contraindication-for-coadministration information or precaution-for-coadministration information in response to a result of the specification.

It is to be noted that, if the medicine coadministration database is recorded in a different apparatus such as the data center 12, then the attribute information generation unit 311 may issue a request to the different apparatus to search for a medicine that is contraindicated in coadministration or precautioned in coadministration through the communication unit 301.

In this manner, the attribute information generation unit 311 successively determines the dispensing dates including the current dispensing date to generate attribute information relating to medicines for each of the dispensing dates.

At step S45, the medication schedule information generation unit 312 generates medication schedule information in regard to the medicines whose attribute information is generated on the basis of the dispensing date, prescription period, prescription amount, medicine type, taking report information and so forth of the medicines included in the medication history data. At this time, for example, the medication schedule information generation unit 312 generates medication schedule information displayed in accordance with a display form according to the degree of a medicinal effect (duration) or a taking method of the medicine as described hereinabove with reference to FIG. 8 or 9.

Further, where a plurality of medicines correspond to one piece of attribute information on one dispensing date, medication schedule information is generated on the basis of a medicine having a high priority degree such as to generate medication schedule information on the basis of a medicine whose medication period is longest.

At step S46, the plot position specification unit 313 specifies a plot position of the dispensing date mark 82 of each dispensing date on the time axis 81 and a display form of the dispensing date mark 82 on each dispensing date on the basis of the medication history data. Here, not only a dispensing date on which attribute information to a medicine is generated but also a plot position and a display form of a dispensing date mark 82 on all dispensing dates are specified.

For example, the plot position specification unit 313 uses, for a dispensing date on which a predetermined number of pieces of attribute information or more are generated, a display form in which an additional information mark is displayed, but uses, for a dispensing date on which a number of pieces of attribute information less than the predetermined number are generated, another display form in which an additional information mark is not displayed. Further, the plot position of a dispensing date mark is set to the position of the date of the dispensing date of the medicine indicated by the medication history data on the time axis 81.

At step S47, the control unit 304 generates display data for the medication history plot displaying region 53 on the basis of the attribute information obtained at step S44, the medication schedule information obtained at step S45 and a specification result of the plot position and the display form obtained at step S46.

At this time, in regard to a dispensing date for which the additional information mark is displayed in the dispensing date mark, the control unit 304 selects attribute information and medication schedule information to be displayed in the medication history plot displaying region 53 on the basis of the attribute priority degree information recorded in the recording unit 305.

Consequently, display data for causing the medication history plot displaying region 53, in which medication history information of the dispensing dates including the dispensing date mark 82, attribute information, medication schedule information and so forth is plot-displayed in a time series on the time axis 81, to be displayed is obtained.

At step S48, the control unit 304 generates display data for the medication history displaying region 54 on the basis of the medication history data and the attribute information obtained at step S44.

For example, the control unit 304 generates display data for the medication history displaying region 54 by generating detail medication history information on the basis of the medication history data and the attribute information in regard to the current dispensing date and the dispensing date selected by the pointer 86.

At this time, the control unit 304 calculates the remaining amount of each medicine from the prescription information or the dispensing history information included in the medication history data, more particularly, from the dispensing date of the medicine, the prescription period of the medicine, the prescription dose of the medicine, the taking method of the medicine, the taking report information and so forth. Further, where the taking report information includes information indicative of a remaining amount of a medicine, the remaining amount indicated by the information is determined as the remaining amount of the medicine.

It is to be noted that, although the dispensing date selected by the pointer 86 is selected by a user or the like, when the position of the pointer 86 is not designated by a user or the like, the dispensing date determined in accordance with a specific condition such as, for example, a dispensing date immediately preceding to the current dispensing date is determined as the dispensing date selected by the pointer 86.

Further, part or the entirety of attribute information and information relating to medicines such as medicine names may be displayed in the detail medication history information displaying region 92 only in regard to medicines having medication periods overlapping with those of the medicines dispensed on the dispensing date selected by the pointer 85 as described above.

In such a case as just described, the control unit 304 calculates a medication period for each medicine, for example, similarly as at step S45, specifies, from a result of the calculation, medicines whose medication period overlaps with the medication periods of the medicines dispensed on the dispensing date selected by the pointer 85 as a display target, and generates display data.

At step S49, the control unit 304 generates display data for the medication history display image 41 on the basis of the display data personal information displaying region 51, display data for the attribute information selection region 52, display data for the medication history plot displaying region 53 and display data for the medication history displaying region 54 obtained by the processes described above.

At step S50, the display controlling unit 314 supplies the display data for the medication history display image 41 to the display unit 303 such that the medication history display image 41 is displayed, and then the medication history displaying process is ended. Consequently, the medication history display image 41 depicted, for example, in FIG. 4 is displayed on the display unit 303.

The franchise store terminal apparatus 21 generates display data for the medication history display image 41 on the basis of the acquired medication history data and the newly generated medication history data and causes the medication history display image 41 to be displayed in such a manner as described above.

At this time, by displaying medication history information of other dispensing dates in a display form according to information relating to the medicines of the current dispensing date noticed specifically and selection attributes, a user, a pharmacist or the like who sees the medication history display image 41 can more rapidly and intuitively grasp necessary information such as a relation between the medicines on the current dispensing date and the medicines on other dispensing dates.

In particular, by displaying or not displaying same medicine information, contraindication-for-coadministration information, precaution-for-coadministration information or the like as attribute information configuring the medication history information on other dispensing dates, for example, in response to information relating to the medicines on the current dispensing date and a selection attribute, a greater amount of information relating to the medicines can be grasped more rapidly and intuitively.

### <Description of Detail Displaying Process>

Further, if a selection of attributes of attribute information to be displayed is made again or a dispensing date is re-selected through movement of the pointer 86 by a user, a pharmacist or the like in a state in which the medication history display image 41 is displayed on the display unit 303, then the franchise store terminal apparatus 21 re-generates the medication history display image 41 in response to such an operation as just described to update the display of the medication history display image 41.

Further, if an instruction to display the detail display image 151 is issued by selection of the detail button 71 by a user, a pharmacist or the like in a state in which the medication history display image 41 is displayed, then the franchise store terminal apparatus 21 performs a detail displaying process to cause the detail display image 151 to be displayed. In the following, the detail displaying process by the franchise store terminal apparatus 21 is described with reference to a flow chart of FIG. 13.

It is to be noted that, at a point of time at which this detail displaying process is performed, the medication history data acquired at step S13 of FIG. 11, medication history data generated at step S14 and attribute information or medication schedule information obtained by the medication history displaying process described hereinabove with reference to FIG. 12 are retained in the control unit 304 of the franchise store terminal apparatus 21.

At step S71, the control unit 304 specifies a remaining medicine situation of medicines of the medication history information to be displayed in the medication history plot displaying region 162.

For example, the control unit 304 specifies the remaining medicine situation of the medicines by using a result of the calculation of the remaining amount of the medicines at step S48 of FIG. 12 or by performing a process similar to that in the case of at step S48 as occasion demands. It is to be noted that the remaining amount of the medicines may not necessarily be displayed in the detail display image 151, and in such a case as just described, the process at step S71 is not performed.

At step S72, the plot position specification unit 313 specifies the plot position on the time axis 81 of the dispensing date mark 167 on each dispensing date and the display form of the dispensing date mark 167 on each dispensing date on the basis of the medication history data and the remaining medicine situation specified at step S71.

In this case, the plot positions of the dispensing date mark 167, namely, the plot positions of the medication history information, are same as the plot positions determined at step S46 of FIG. 12. Further, the plot position specification unit 313 sets, in response to the remaining medicine situation, the display form of the dispensing date mark 167 at each dispensing date to one of the display forms of "no remaining medicine," "remaining medicine present" and "any other case" described hereinabove with reference to FIG. 7.

At step S73, the control unit 304 generates display data for the detail display image 151 on the basis of the plot positions and the display forms specified at step S72, attribute information obtained at step S44 of FIG. 12 and medication schedule information obtained at step S45.

Consequently, display data for causing the detail display image 151, in which the medication history information of the dispensing dates including the dispensing date marks 167, attribute information, medication schedule information and so forth are plot-displayed in a time series on the time axis 81, are obtained.

At step S74, the display controlling unit 314 supplies the display data for the detail display image 151 to the display unit 303 such that the detail display image 151 is displayed, and then the detail displaying process is ended. Consequently, for example, the detail display image 151 depicted in FIG. 7 is displayed on the display unit 303.

The franchise store terminal apparatus 21 generates display data for the detail display image 151 on the basis of the acquired medication history data and the newly generated medication history data and causes the detail display image 151 to be displayed in such a manner as described above.

At this time, similarly as in the case of the medication history display image 41, by displaying medication history information of other dispensing dates in a display form according to information relating to the medicines of the current dispensing date noticed specifically and selection attributes, it is possible to more rapidly and intuitively grasp necessary information.

It is to be noted that, while the foregoing description is directed to an example in which the franchise store terminal apparatus 21 generates the medication history display image 41 or the detail display image 151, the medication history display image 41 and the detail display image 151 may otherwise be generated by the data center 12 or the portable terminal apparatus 14.

For example, there is a case in which the portable terminal apparatus 14 accesses the data center 12 to enjoy a service regarding a medicine notebook or another case in which a user utilizes a personal computer in its own home to access the data center 12 or the like and enjoy a service relating to a medicine notebook on a web page. In such a case as just described, the data center 12 or the like may perform processes similar to the processes described hereinabove with reference to FIG. 12 or FIG. 13 to generate a medication history display image 41 or a detail display image 151 and transmit the medication history display image 41 or the detail display image 151 to the portable terminal apparatus 14 or the personal computer of the user.

Further, also there is a case in which the portable terminal apparatus 14 can access the data center 12 to acquire medication history data. In such a case as just described, the portable terminal apparatus 14 may use the acquired medication history data to perform processes similar to the processes described hereinabove with reference to FIG. 12 or FIG. 13 to generate and display a medication history display image 41 or a detail display image 151.

### <Second Embodiment>

### <Display Example of Detail Display Image>

It is to be noted that, in the foregoing, an example is described in which only attribute information relating to medicines is displayed as attribute information to be displayed on the medication history display image 41 or the detail display image 151. However, information relating to a result of measurement in which a patient itself is a target, information relating to a result of inspection of components in the body of the patient, information relating to the substance of the meals of the patient and so forth may be displayed as the attribute information.

In the following description, where side effect information, in-medication information, same medicine information, contraindication-for-coadministration information and precaution-for-coadministration information that are attribute information relating to a medicine need not distinguished specifically from each other, they are referred to also as medicine attribute information.

Further, not only the medicine attribute information but also measurement value attribute information, inspection value attribute information and meal substance attribute information are available as the attribute information. These kinds of attribute information are, for example, information obtained from patient information that is recorded in an associated relation with a personal identification ID in the data center 12 and is information relating to the patient (user) itself. Here, the information itself recorded as the patient information is displayed as the attribute information.

More particularly, attribute information indicating a result of measurement in which a patient itself is a target such as a systolic blood pressure, a diastolic blood pressure, a pulse rate, a weight and a temperature of the patient is included, for example, in the measurement value attribute information.

Here, for example, systolic blood pressure information indicating a systolic blood pressure of the patient, diastolic blood pressure information indicating a diastolic blood pressure of the patient, body weight information indicating a body weight of the patient and body temperature information indicating a body temperature of the patient are displayed as measurement value attribute information on the detail display image 151 as occasion demands. It is to be noted that, in the following expression, attributes to which the systolic blood pressure information, diastolic blood pressure information, body weight information and body temperature information belong are especially referred to also as systolic blood pressure attribute, diastolic blood pressure attribute, body weight attribute and body temperature attribute, respectively.

Where measurement value attribute information is displayed on the detail display image 151, information depicted, for example, in FIG. 14 is recorded as patient information in the recording unit 24 of the data center 12.

In the example depicted in FIG. 14, a personal identification ID and patient information that includes a measurement date and time, a measurement value type and a measurement value are recorded in an associated relation with each other.

Here, the "measurement value type" is information indicating what kind of information the measurement information such as a systolic blood pressure, a diastolic blood pressure, a pulse rate or a body weight is, and the "measurement date and time" indicates a date and time at which the measurement is performed. Further, the "measurement value" indicates a measurement result of information of the type indicated by the "measurement value type."

In this example, the information of the type indicated by the measurement value type is a type (attribute) of the attribute information and the measurement value is attribute information.

Further, for example, the inspection value attribute information includes attribute information indicative of a result of inspection of various components such as sodium, potassium, calcium, inorganic phosphorus, uric acid, cholesterol and triglyceride, performed for the patient determined as a target.

In this case, for example, in the data center 12, information indicative of a measurement result of a systolic blood pressure or the like or information indicative of an inspection result of various components where the patient is a target may be included in patient information recorded in an associated relation with a personal identification ID.

Further, for example, the meal substance attribute information includes attribute information indicating the meal substance of the patient. The meal substance here indicates, for example, food that has an influence when it is combined with a medicine. More particularly, it is known that, when, for example, a grapefruit or fermented soybeans are taken in, if a specific medicine is taken, then it has a bad influence on the body.

Therefore, in order to arouse attention, for example, on a day on which a grapefruit, fermented soybeans or the like, which has an influence, for example, when it is combined with a medicine, is taken in (eaten or drunken), meal substance attribute information indicative of the meal substance is plot-displayed in the medication history plot displaying region 162. It is to be noted that displaying of an alert mark or the like for urging a notice may be performed when a notice is necessary for a combination of medicine in each dispensing date and meal substance.

It is to be noted that, as the information indicative of the meal substance, information inputted by a patient itself, nursing care staff or the like when hearing is performed, for example, by a medical employee or the like may be included in the patient information.

Where meal substance attribute information is displayed on the detail display image 151, information depicted, for example, in FIG. 15 is recorded as the patient information in the recording unit 24 of the data center 12.

In the example depicted in FIG. 15, a personal identification ID and patient information including an inputting date and time and the input substance are recorded in an associated relation with each other.

Here, the "input substance" is information indicative of the inputted meal substance and, here, from within the meal substance, especially a piece of foodstuff for which a precaution is required or the like is inputted. Further, the "inputting date" indicates a date on which information indicated in the "input substance" is inputted and, especially here, the inputting day indicates a date and time at which the meal substance indicated in the input substance is provided, namely, a date and time at which the meal is had.

In this example, information indicated by the input substance is the meal substance attribute information.

In this manner, where not only medicine attribute information but also measurement value attribute information, inspection value attribute information and meal substance attribute information can be displayed as the attribute information, the detail display image 151 depicted in FIG. 16 is displayed, for example, on the display unit 303. It is to be noted that, in FIG. 16, elements corresponding to those in FIG. 7 are denoted by the same reference signs and description of them is omitted suitably.

In the detail display image 151 depicted in FIG. 16, a tab for selecting an attribute of attribute information to be displayed in the medication history plot displaying region 162 is newly provided in the attribute information selection region 161 from among pieces of attribute information including medicine attribute information, measurement value attribute information, inspection value attribute information and meal substance attribute information.

In particular, a medicine tab 341 for selecting the attribute relating to the medicine attribute information, a measurement value 342 for selecting the attribute relating to the measurement value attribute information, an inspection value 343 for selecting the attribute relating to the inspection value attribute information and a meal substance tab 344 for selecting the attribute relating to the meal substance attribute information are provided in the attribute information selection region 161.

A user, a pharmacist or the like will select one of the tabs described above to cause a check box for selecting an attribute corresponding to the selected tab to be displayed in the attribute information selection region 161.

In this example, the measurement value tab 342 is selected, and a check box 345 for selecting the systolic blood pressure attribute, a check box 346 for selecting the diastolic blood pressure attribute, a check box 347 for selecting the body weight attribute and a check box 348 for selecting the body temperature attribute are displayed in the attribute information selection region 161.

A user, a pharmacist or the like will operate a check box to cause a checkmark in the check box to select one of the attributes of the measurement value attribute information.

Further, for example, if a state in which the medicine tab 341 is selected is entered, then, for example, a side effect mark 61 to a precaution-for-coadministration mark 65 and check boxes 66 to 70 depicted in FIG. 7 are displayed in the attribute information selection region 161.

Further, in the medication history plot displaying region 162, a time axis 81 is displayed and a dispensing date mark 167 for each dispensing date is plot-displayed on the time axis 81.

Furthermore, a region for displaying attribute information including the medicine attribute information, measurement value attribute information, inspection value attribute information and meal substance attribute information is provided at the lower side in the figure of the time axis 81 in the medication history plot displaying region 162.

In this example, a region 351 for displaying medicine attribute information, a region 352 for displaying systolic blood pressure information as measurement value attribute information and a region 353 for displaying diastolic blood pressure information as measurement value attribute information are provided.

It is to be noted that, although the medication history plot displaying region 162 has provided therein regions for displaying other pieces of attribute information, since, in the present example, such attribute information cannot be displayed fully, the regions are in an invisible state. If a user or the like operates a scroll button 354 or the like to scroll the medication history plot displaying region 162 upwardly or downwardly in the figure, then the regions for displaying the other attribute information become visible.

In the region 351, attribute information classified into the medicine attribute information such as the in-medication information 83-1, the medication schedule information 84-1 and so forth and medication schedule information are displayed similarly as in the example depicted in FIG. 7.

Further, in the region 352, a graph 355 indicative of a systolic blood pressure of a user (patient) measured on each date on the time axis 81 is displayed. In particular, a round mark on the graph 355 indicates a measurement result of a systolic blood pressure of the user (patient) measured on the date of the round mark.

Especially, in the graph 355, as regards a date on which the measurement result of the systolic blood pressure is outside a range of a normal value, a round mark indicative of a measurement result on the date is displayed in a display form different from that of a round mark indicative of a measurement result of the other dates, as indicated by an arrow mark A31, for example. In other words, by displaying the round mark in a display form different from that on the other dates, an alert display (warning display) is given to call out attention. Further, in this example, on a date on which the measurement result is outside the range of a normal value, also a numerical value "153" inactive of a measurement result of the systolic blood pressure is displayed.

It is to be noted that, not only with regard to the systolic blood pressure but also with regard to the diastolic blood pressure information, body weight information, body temperature information and inspection value attribute information, for a date on which the measurement value or inspection value is outside the range of a normal value, a measurement value is displayed together with an alert display similarly as in the case of the systolic blood pressure information.

Further, although an alert display is performed here by making a display form of a measurement result different and further displaying a numerical value, an alert display may be performed by any other method such as a popup display or the like.

By displaying not only attribute information relating to medicines but also attribute information relating to a measurement value, an inspection value, the meal substance and so forth in this manner, a user, a pharmacist or the like who sees the information can cause various information to be displayed at the same time so as to make it possible to visually compare and refer to them. As a result, the user, pharmacist or the like can more rapidly and intuitively grasp necessary information.

Where such a detail display image 151 as depicted in FIG. 16 is displayed, for example, in the medication history data acquisition process described hereinabove with reference to FIG. 11, the communication unit 301 at step S13 acquires medication history data and patient information registered in an associated relation with a personal identification ID from the data center 12. Further, for example, in the detail displaying process described hereinabove with reference to FIG. 13, the attribute information generation unit 311 generates measurement value attribute information, inspection value attribute information and meal substance attribute information on the basis of information indicative of measurement results, information indicative of inspection results of various components and information indicative of the meal substance included in the acquired patient information.

### <Third Embodiment>

### <Display Example of Detail Display Image>

Further, when the detail display image 151 or the like is displayed on the franchise store terminal apparatus 21 or the like such that a pharmacist performs confirmation of medicines prescribed for a user (patient), a doubtful point sometimes occurs in regard to an interaction or a side effect of a prescribed medicine or a taking coadministration of overlapping medicines.

It is assumed that, in such a case as just described, a pharmacist issues a doubtful point inquiry to a doctor by whom medicines are prescribed to a user and inputs information indicative of a result of the inquiry as part of medication history data and the inputted information is transmitted from the franchise store terminal apparatus 21 to and registered into the data center 12. Here, information indicative of a result of a doubtful point inquiry is such information that an approval for dispensing in regard to the doubtful point is obtained or the like.

In this case, the recording unit 24 of the data center 12 has recorded therein, for example, information depicted in FIG. 17 as information indicative of a result of the doubtful point inquiry.

In the example depicted in FIG. 17, information including a prescription ID, an inquiry date, a medicine name, an inquiry destination medical facility and inquiry contents is the information indicative of a result of the doubtful point inquiry.

Here, the "prescription ID" is information indicative of a prescription in which a medicine about which the inquiry is made is prescribed; the "inquiry date" indicates the date on which the inquiry is issued; and the "medicine name" indicates a result of the medicine about which the inquiry is issued. Further, the "inquiry destination medical facility" indicates a medical facility of an inquiry destination, namely, a medical facility from which the prescription indicated by the "prescription ID" is issued, and the "inquiry substance" indicates the substance of the inquiry issued about the medicine.

Where the medication history data includes information indicative of such a result of a doubtful point inquiry that an approval for dispensing is obtained in this manner, an inquired mark representing that the doubtful point is inquired already may be displayed in the medication history plot displaying region 162 on the detail display image 151, for example, as depicted in FIG. 18. It is to be noted that, in FIG. 18, corresponding portions to those in FIG. 16 are denoted by the same reference signs, and description of them is suitably omitted.

In the example of FIG. 18, contraindication-for-coadministration information 381 as attribute information and medication schedule information 382 of a medicine corresponding to the contraindication-for-coadministration information 381 are displayed at a dispensing date indicated by a dispensing date mark 167-7. Then, in this example, it is assumed that, as regards the medicine corresponding to the contraindication-for-coadministration information 381, information that a doubtful point has been inquired is included in the medication history data.

In such a case as described above, the attribute information generation unit 311 generates, at step S44 of FIG. 12, also inquiry completion mark 383 on the basis of the information that the doubtful point included in the medication history data has been inquired already. Then at step S73 of FIG. 13, display data for the detail display image 151 in which also the inquiry completion mark 383 depicted in FIG. 18 is displayed together with the contraindication-for-coadministration information 381 is generated.

Consequently, at step S74 of FIG. 13, the medication history plot displaying region 162 depicted in FIG. 18 is displayed in the detail display image 151. If information regarding a result when a doubtful point inquiry is made is included in the medication history data in this manner, then if also the information indicative of the inquiry result is displayed, then a pharmacist or the like can acquire a greater amount of information.

Further, if a user, a pharmacist or the like operates (selects) the inquiry completion mark 383 in a state in which, for example, the display depicted in FIG. 18 is performed, then detailed information relating to doubtful point inquiries such as the inquiry date of the doubtful point, a hospital or a doctor name of an inquiry destination, the inquiry substance and so forth may be displayed in a different region, on a different screen image or the like.

In this case, for example, the control unit 304 generates display data of detail information relating to the doubtful point inquiry on the basis of information indicative of a result of the doubtful point inquiry included in the medication history data and causes an image based on the display data, namely, detail information regarding the doubtful point inquiry, in a region different from the medication history plot displaying region 162 or in a different screen image. At this time, the pharmacist or the like may further perform inputting of information regarding the doubtful point inquiry.

It is to be noted that, although an example in which information relating to a doubtful point inquiry, namely, an inquiry completion mark 383, is displayed in the detail display image 151 here, information relating to the doubtful point inquiry may be displayed also in the medication history display image 41.

### <Fourth Embodiment>

### <Display Example of Detail Display Image>

Further, where medicines corresponding to attribute information have some relation to each other, the medication history display image 41 or the detail display image 151 may provide such a display from which a relation between the medicines can be recognized.

Medicines are available which are prescribed at the same time in combination in such a case that an intestinal regulator is prescribed for an antibiotic like a Cravit tablet (registered trademark) that is an antibiotic and a Biofermin tablet (registered trademark) that is an intestinal regulator. For such medicines that have a relation to each other and are to be taken at the same time, relation marks indicative of such a relation as just described may be displayed.

Further, as regards medicines having a relation in taking date in that, after taking of a predetermined one of the medicines ends, taking of the other particular one of the medicines is started, a taking date relation mark indicative of such a relation in taking date as just described may be displayed.

It is to be noted that information for specifying a relation between medicines may be included, for example, as part of medication history data. For example, when a doctor or the like issues a prescription, when a pharmacist or the like performs dispensing or in a like case, information indicative of a relation between medicines may be inputted as medication history data. In this case, the information inputted by the doctor, pharmacist or the like is transmitted as medication history data from the franchise store terminal apparatus 21 to and recorded into the data center 12.

In particular, for example, information depicted in FIG. 19 is recorded as information for specifying a relation between medicines into the recording unit 24 of the data center 12 in an associated relation with dispensing history information or the like.

In the example depicted in FIG. 19, a relation source prescription ID, a name of a medicine, a relation destination prescription ID, a name of a relation destination medicine and information indicative of relation details is information for specifying a relation between medicines.

Here, the "name of medicine" indicates a name of a medicine for which a relation mark or a taking date relation mark is to be displayed, and the "relation source prescription ID" is information indicative of a prescription in which the medicine is prescribed. Meanwhile, the "name of relation destination medicine" indicates a name of a medicine having a relation to the medicine indicated by the "name of medicine" described above, namely, a name of a medicine to be taken at the same time or of a medicine having a relation in taking date, and the "relation destination prescription ID" is information indicative of a prescription in which the medicine having the relation is prescribed.

Further, the "relation details" indicates detailed information of the relation between the medicine indicated by the "name of medicine" and the medicine indicated by the "name of relation destination medicine."

In particular, "medicate at same time" indicates that the medicines are to be taken simultaneously. In short, this indicates that it becomes a displaying target of a relation mark. Further, "medicate after end of taking of relation source" indicates that, after taking of the medicine indicated by the "name of medicine" ends, taking of the medicine indicated by the "name of relation destination medicine" is started. In short, this indicates that it becomes a displaying target of a taking date relation mark.

Where a relation mark or a taking date relation mark is displayed in the detail display image 151 on the basis of the information depicted in FIG. 19 and so forth in this manner, the display of the medication history plot displaying region 162 in the detail display image 151 is, for example, such a display as depicted in FIG. 20. It is to be noted that, in FIG. 20, portions corresponding to those in the case of FIG. 18 are denoted by the same reference signs, and description of them is suitably omitted.

In the example depicted in FIG. 20, the relation between a medicine corresponding to the side effect information 83-4 and a medicine corresponding to the contraindication-for-coadministration information 381 is such a relation that, after taking of the medicine corresponding to the side effect information 83-4 ends, taking of the medicine corresponding to the contraindication-for-coadministration information 381 is started.

Therefore, an arrow mark that has a start point at the right end in the figure of the medication schedule information 84-4 displayed together with the side effect information 83-4, namely, at the completion date of the medication and is directed toward the contraindication-for-coadministration information 381, more particularly toward the left end in FIG. 20 of the medication schedule information 382 displayed together with the contraindication-for-coadministration information 381, namely, toward the taking starting date, is displayed as a taking date relation mark 411.

Accordingly, for example, the medicine corresponding to the side effect information 83-4 corresponds to "Loxonin 50 mg tablet" of the example depicted in FIG. 19, and the medicine corresponding to the contraindication-for-coadministration information 381 corresponds to the "Lobu 50 mg tablet" in the example depicted in FIG. 19.

If a user, a pharmacist or the like sees the taking date relation mark 411, then it can rapidly and intuitively grasp that, after taking of the medicine corresponding to the side effect information 83-4 ends, taking of the medicine corresponding to the contraindication-for-coadministration information 381 is to be started.

Further, in the example depicted in FIG. 20, contraindication-for-coadministration information 412 and medication schedule information 413 as well as precaution-for-coadministration information 414 and medication schedule information 415 are displayed at the position of the current dispensing date, and the medicines corresponding to the attribute information are medicines prescribed simultaneously in combination. In other words, the medicines have such a relation as that of the Cravit tablet and the Biofermin tablet or such a relation as that of the "Clarith tablet 50" and "Biofermin R tablet" depicted in the example of FIG. 19.

Therefore, a line segment interconnecting the contraindication-for-coadministration information 412 and the precaution-for-coadministration information 414 here is displayed as a relation mark 416. If a user, a pharmacist or the like sees this relation mark 416, it can rapidly and intuitively grasp that the medicines corresponding to the pieces of attribute information linked to each other by the relation mark 416 are medicines having a relation therebetween.

Where a display of such a taking date relation mark 411 or a relation mark 416 is to be performed, the control unit 304 specifies, in the detail displaying process described hereinabove with reference to FIG. 13, a relation between the medicines, in particular, presence or absence of a relation between the medicines, on the basis of the medication history data, and generates display data for the detail display image 151 in which the taking date relation mark 411 or the relation mark 416 is to be displayed on the basis of a result of the specification.

It is to be noted that, although an example in which the taking date relation mark 411 or the relation mark 416 is displayed in the detail display image 151 is described here, such marks may be displayed also in the medication history display image 41. Alternatively, setting information indicative of a medicine having a relation as attribute information of one attribute, it may be made possible to select the attribute as a selection attribute in the attribute information selection region 52 or the attribute information selection region 161.

### <Fifth Embodiment>

### <Display Example of Detail Display Image>

Further, depending upon a medicine, there are a case in which a medicine holiday is provided like Rheumatrex (registered trademark), another case in which an instruction is sometimes issued to take such a medicine as a conscription medicine after lapse of a taking time period, namely, to provide a medicine washout period and so forth. Therefore, where a medicine requires a washout period, a medicine washout time mark indicative of a washout time period of the medicine may be displayed on the medication history display image 41 or the detail display image 151.

In such a case as described above, a medicine washout time mark is displayed in a medication history plot displaying region 162 on the detail display image 151, for example, as depicted in FIG. 21. It is to be noted that, in FIG. 21, portions corresponding to those in the case of FIG. 16 are denoted by the same reference signs, and description of them is suitably omitted.

In this example, a medicine corresponding to same medicine information 168-1 is a medicine that requires medicine washout time (medicine washout period), and a medicine holiday mark 441 is displayed along the medication schedule information 169-1 together with the same medicine information 168-1.

Here, a mark including a dotted line indicative of medicine washout and characters indicative of a medicine washout period "medicine washout 8 hours," displayed along the medication schedule information 169-1 indicative of a taking period of the medicine is displayed as the medicine holiday mark 441.

Further, a medicine corresponding to the contraindication-for-coadministration information 83-3 is a medicine that requires medicine washout time, and a medicine washout time mark 442 is displayed along the medication schedule information 84-3 together with the contraindication-for-coadministration information 83-3. Here, a mark including a dotted line indicative of medicine washout and characters indicative of a medicine washout period "medicine washout 6 days," displayed along the medication schedule information 84-3 indicative of a taking period of the medicine is displayed as a medicine washout time mark 442.

By displaying such a medicine holiday mark 441 or a medicine washout time mark 442 as described above, a user can rapidly and intuitively grasp that washout time is required upon medication of the medicine. Further, also a pharmacist or the like can rapidly and intuitively grasp that medicine washout is required upon medication instruction.

When a display of the medicine washout time mark is to be performed, the control unit 304 specifies, on the basis of a medicine name and so forth included in medication history data, whether or not there is the necessity to display the medicine washout time mark for each medicine and generates display data for a detail display image 151 on the basis of a result of the specification in the detail displaying process described hereinabove with reference to FIG. 13.

It is to be noted that, while an example in which the medicine washout time mark is displayed in the detail display image 151 is described here, the medicine washout time mark may be displayed also in the medication history display image 41. Further, using information indicative of a medicine that requires medicine washout as attribute information of one attribute, it may be made possible to select the attribute as a selection attribute in the attribute information selection region 52 or the attribute information selection region 161.

Further, according to the present technology, naturally it is possible to suitably combine the first embodiment to the fifth embodiment described hereinabove.

Further, while, in the foregoing description, an example in which a medicine coadministration database is recorded in the franchise store terminal apparatus 21 is described, the medicine coadministration database may otherwise be recorded in the data center 12 or some other apparatus.

Further, while the foregoing description is directed to an example in which personal information is acquired from the IC card 13 and medication history information and so forth are acquired from the data center 12, such information as personal information and medication history data and so forth may be recorded anywhere and may be recorded in a form in which it is divided to a plurality of parts. Similarly, also various processes such as the medication history displaying process may be performed by the franchise store terminal apparatus 21 and may be performed by any apparatus such as the portable terminal apparatus 14 or the data center 12, and at least from the medicine associated with a specific person and a medication period of the medicine, information relating to the medicine is to be displayed in a time series.

Incidentally, while a series of processes may be executed by hardware, it may otherwise be executed by software. Where the series of processes is executed by software, a program that constructs the software is installed into a computer for exclusive use or the like. a program which constructs the software is installed into a computer. Here, the computer includes a computer incorporated in hardware for exclusive use and, for example, a personal computer for universal use that can execute various functions by installing various programs.

FIG. 22 is a block diagram depicting a configuration example hardware of a computer that executes the series of processes described above in accordance with a program.

In the computer, a CPU (Central Processing Unit) 501, a ROM (Read Only Memory) 502 and a RAM (Random Access Memory) 503 are connected to each other by a bus 504.

To the bus 504, an input/output interface 505 is connected further. To the input/output interface 505, an inputting unit 506, an outputting unit 507, a recording unit 508, a communication unit 509 and a drive 510 are connected.

The inputting unit 506 includes a keyboard, a mouse, a microphone, an image pickup element and so forth. The outputting unit 507 includes a display unit, a speaker and so forth. The recording unit 508 includes a hard disk, a nonvolatile memory and so forth. The communication unit 509 includes a network interface and so forth. The drive 510 drives a removable recording medium 511 such as a magnetic disk, an optical disk, a magneto-optical disk or a semiconductor memory.

In the computer configured in such a manner as described above, the CPU 501 loads a program recorded, for example, in the recording unit 508 into the RAM 503 through the input/output interface 505 and the bus 504 and executes the program to perform the series of processes described hereinabove.

The program to be executed by the computer (CPU 501) can be recorded into and provided together with the removable recording medium 511, for example, as a package medium. Further, the program can be provided through a wired or wireless transmission medium such as a local area network, the Internet or a digital satellite broadcast.

In the computer, the program can be installed into the recording unit 508 through the input/output interface 505 by loading the removable recording medium 511 into the drive 510. Further, the program can be received through a wired or wireless transmission medium by the communication unit 509 and installed into the recording unit 508. Further, it is possible to install the program into the ROM 502 or the recording unit 508 in advance.

It is to be noted that the program executed by the computer may be a program in which processes are performed in a time series in the order as described in the present specification or may be a program in which processes are executed in parallel or at necessary timings such as a timing at which the program is called.

Further, the embodiment of the present technology is not limited to the embodiments described above and can be changed in various manners without departing from the subject matter of the present technology.

For example, the present technology can take a configuration for crowd computing by which one function is shared by a plurality of apparatus through a network such that it is processed by cooperation of the plurality of apparatus.

Further, the steps described hereinabove in connection with the flow chars not only can be executed by one apparatus but also can be shared and executed by a plurality of apparatus.

Furthermore, where a plurality of processes are included in one step, the plurality of processes included in the one step not only can be executed by one apparatus but also can be shared and executed by a plurality of apparatus.

Further, the present technology can take such configurations as described below.
(1) An information processing apparatus, including:
   a control unit configured to control such that pieces of medication history information recorded in an associated relation with personal identification information for identifying a patient are displayed in a lined up relation in a time series; in which
   the control unit controls such that the medication history information other than specific medication history information is displayed in a display form according to the specific medication history information of the medication history information.
(2) The information processing apparatus according to (1), in which
   the specific medication history information is information of one or more medicines dispensed, prescribed or taken at a specific point of time.
(3) The information processing apparatus according to (1) or (2), in which
   the medication history information includes information relating to a medicine scheduled to be taken in the future by the patient.
(4) The information processing apparatus according to one of (1) to (3), in which
   a display form according to specific medication history information selected from within the medication history information is a display form based on attribute information relating to coadministration of a medicine included in the specific medication history information and a medicine included in the medication history information other than the specific medication history information.
(5) The information processing apparatus according to (4), in which
   the attribute information includes an attribute relating to contraindication for coadministration and an attribute relating to precaution for coadministration.
(6) The information processing apparatus according to (4), in which,
   where a plurality of attributes different from each other are included in a same dispensing date, the control unit controls such that the attribute having a higher priority degree is displayed preferentially.
(7) The information processing apparatus according to any one of (1) to (6), in which
   the control unit controls such that medication period information is displayed in a bar-like display as the medication history information other than the specific medication history information.
(8) The information processing apparatus according to (7), in which
   the medication period information is information indicative of a medication period calculated from prescription information or dispensing information of the medication history information or a medication period during which a medicine of the medication history information is medicated actually.
(9) The information processing apparatus according to (7), in which
   the bar-like display is a display indicative of a gradual decrease of a medicinal effect of a medicine or a medication amount of the medicine.
(10) The information processing apparatus according to any one of (1) to (9), in which
   the control unit controls such that a remaining number of the medicines of the medication history information is calculated and displayed on the basis of prescription information or dispensing information of the medication history information or a medication period during which a medicine of the medication history information is medicated actually.
(11) The information processing apparatus according to any one of (1) to (10), in which,
   where there is medication history information other than the specific medication history information for medication within a period that overlaps with a medication period of the specific medication history information, the control unit controls such that an alert is displayed.
(12) The information processing apparatus according to any one of (1) to (11), in which
   the control unit controls such that the medication history information is plot-displayed at a position of a dispensing date of the medication history information on a time axis.
(13) The information processing apparatus according to any one of (1) to (12), in which
   the control unit controls such that patient information associated with the personal identification information is displayed.
(14) The information processing apparatus according to (13), in which
   the patient information is information indicative of a measurement value, an inspection value or a meal substance relating to a patient.
(15) The information processing apparatus according to (13) or (14), in which
   the control unit controls such that an alert is displayed on the basis of the medication history information and the patient information.
(16) The information processing apparatus according to any one of (1) to (15), in which
   the control unit controls such that information relating to washout of a medicine of the medication history information is further displayed.
(17) The information processing apparatus according to any one of (1) to (16), further including:
   a display unit configured to display the medication history information on the basis of display data.
(18) The information processing apparatus according to any one of (1) to (17), further including:
   a recording unit configured to record the medication history information in association with the personal identification information.
(19) An information processing method, including the steps of:
   controlling such that pieces of medication history information recorded in an associated relation with personal identification information for identifying a patient are displayed in a lined up relation in a time series, and controlling such that the medication history information other than specific medication history information is displayed in a display form according to the specific medication history information of the medication history information.
(20) A program for causing a computer to execute a process, the process including the steps of:
   controlling such that pieces of medication history information recorded in an associated relation with personal identification information for identifying a patient are displayed in a lined up relation in a time series, and controlling such that the medication history information other than specific medication history information is displayed in a display form according to the specific medication history information of the medication history information.

### [Reference Signs List]

11 Franchise store system, 12 Data center, 21 Franchise store terminal apparatus, 301 Communication unit, 303 Display unit, 304 Control unit, 305 Recording unit, 311 Attribute information generation unit, 312 Medication schedule information generation unit, 313 Plot position specification unit, 314 Display controlling unit

## Claims

1. An information processing apparatus, comprising:
a control unit configured to control such that pieces of medication history information recorded in an associated relation with personal identification information for identifying a patient are displayed in a lined up relation in a time series; wherein
the control unit controls such that the medication history information other than specific medication history information is displayed in a display form according to the specific medication history information of the medication history information.

2. The information processing apparatus according to claim 1, wherein
the specific medication history information is information of one or more medicines dispensed, prescribed or taken at a specific point of time.

3. The information processing apparatus according to claim 1, wherein
the medication history information includes information relating to a medicine scheduled to be taken in the future by the patient.

4. The information processing apparatus according to claim 1, wherein
a display form according to specific medication history information selected from within the medication history information is a display form based on attribute information relating to coadministration of a medicine included in the specific medication history information and a medicine included in the medication history information other than the specific medication history information.

5. The information processing apparatus according to claim 4, wherein
the attribute information includes an attribute relating to contraindication for coadministration and an attribute relating to precaution for coadministration.

6. The information processing apparatus according to claim 4, wherein,
where a plurality of attributes different from each other are included in a same dispensing date, the control unit controls such that the attribute having a higher priority degree is displayed preferentially.

7. The information processing apparatus according to claim 1, wherein
the control unit controls such that medication period information is displayed in a bar-like display as the medication history information other than the specific medication history information.

8. The information processing apparatus according to claim 7, wherein
the medication period information is information indicative of a medication period calculated from prescription information or dispensing information of the medication history information or a medication period during which a medicine of the medication history information is medicated actually.

9. The information processing apparatus according to claim 7, wherein
the bar-like display is a display indicative of a gradual decrease of a medicinal effect of a medicine or a medication amount of the medicine.

10. The information processing apparatus according to claim 1, wherein
the control unit controls such that a remaining number of the medicines of the medication history information is calculated and displayed on the basis of prescription information or dispensing information of the medication history information or a medication period during which a medicine of the medication history information is medicated actually.

11. The information processing apparatus according to claim 1, wherein,
where there is medication history information other than the specific medication history information for medication within a period that overlaps with a medication period of the specific medication history information, the control unit controls such that an alert is displayed.

12. The information processing apparatus according to claim 1, wherein
the control unit controls such that the medication history information is plot-displayed at a position of a dispensing date of the medication history information on a time axis.

13. The information processing apparatus according to claim 1, wherein
the control unit controls such that patient information associated with the personal identification information is displayed.

14. The information processing apparatus according to claim 13, wherein
the patient information is information indicative of a measurement value, an inspection value or a meal substance relating to a patient.

15. The information processing apparatus according to claim 13, wherein
the control unit controls such that an alert is displayed on the basis of the medication history information and the patient information.

16. The information processing apparatus according to claim 1, wherein
the control unit controls such that information relating to washout of a medicine of the medication history information is further displayed.

17. The information processing apparatus according to claim 1, further comprising:
a display unit configured to display the medication history information on the basis of display data.

18. The information processing apparatus according to claim 1, further comprising:
a recording unit configured to record the medication history information in association with the personal identification information.

19. An information processing method, comprising the steps of:
controlling such that pieces of medication history information recorded in an associated relation with personal identification information for identifying a patient are displayed in a lined up relation in a time series, and controlling such that the medication history information other than specific medication history information is displayed in a display form according to the specific medication history information of the medication history information.

20. A program for causing a computer to execute a process, the process comprising the steps of:
controlling such that pieces of medication history information recorded in an associated relation with personal identification information for identifying a patient are displayed in a lined up relation in a time series, and controlling such that the medication history information other than specific medication history information is displayed in a display form according to the specific medication history information of the medication history information.
